# EUROPEAN PATENT SPECIFICATION

(11) **EP 2 600 937 B1**
(45) Date of publication and mention of the grant of the patent: **17.01.2024**
(21) Application number: 11814906.1
(22) Date of filing: 02.08.2011
(51) Int. Cl.: A61N 7/00, A61N 7/02, A61B 18/00, A61B 8/14, A61B 90/00, A61B 17/00, A61B 8/00, A61B 8/08

(54) **SYSTEMS FOR TREATING ACUTE AND/OR CHRONIC INJURIES IN SOFT TISSUE**
SYSTEME ZUR BEHANDLUNG AKUTER UND / ODER CHRONISCHER VERLETZUNGEN DER WEICHTEILE
SYSTÈMES DE TRAITEMENT DE LÉSIONS AIGUËS ET/OU CHRONIQUES DANS DES TISSUS MOUS

(30) Priority: 02.08.2010 US 370095 P; 02.08.2010 US 369782 P; 02.08.2010 US 369793 P; 02.08.2010 US 369806 P
(43) Date of publication of application: 12.06.2013
(73) Proprietor: Guided Therapy Systems, L.L.C., Scottsdale, AZ 85251 (US)
(72) Inventor: SLAYTON, Michael, H., Phoenix, AZ 85018 (US); BARTHE, Peter, G., Phoenix, AZ 85048 (US)
(74) Representative: Maiwald GmbH
(86) International application number: PCT/US2011/001366
(87) International publication number: WO 2012/018390

(56) References cited:
- WO-A1-2006/042201
- WO-A1-2009/149390
- US-A1- 2002 165 529
- US-A1- 2006 122 508
- US-A1- 2006 184 071
- US-A1- 2007 167 709
- US-A1- 2007 239 079
- US-A1- 2008 071 255
- US-A1- 2008 281 237
- US-A1- 2009 253 988
- US-A1- 2009 254 008

## Description

### BACKGROUND

Subcutaneous tissues such as, muscles, tendons, ligaments and cartilage, are important connective tissues that provide force and motion, non-voluntary motion, anchoring, stability, and support among other functions. These tissues are prone to wear and injury due to participation in sports or other daily activities which put stress on these tissues.

Inflammation is a response of a tissue to injury and is characterized by increased blood flow to the tissue causing increased temperature, redness, swelling, and pain. Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process.
Document WO 2009/149390 A1 relates to a dermatological cosmetic treatment and imaging system. The system includes a hand wand with at least one finger activated controller and a removable emitter-receiver module having an ultrasound transducer. The emitter-receiver module has a position encoder, such as a magnet, connected to the transducer and a sensor, such as a Hall sensor, connected to the stationary emitter/receiver housing via a circuit board. The position encoder and the position sensor act as a sensor for determining a transducer home position and/or movement.
Document WO 2006/042201 A1 relates to a system for non-invasive tissue treatment. The system is configured to treat tissue regions, such as the SMAS region, photoaged tissue, acne sebaceous glands and/or sweat glands. First, the region of interest is imaged for localization of the treatment area and surrounding structures. Then, ultrasound energy is delivered to achieve the desired therapeutic effect. Then, the treatment area is monitored before, during and after therapy to plan and assess the results and/or to provide feedback.
Document US 2009/0254008 A1 relates to a system for delivering ultrasonic treatment to a subject. One or more waveform generators, one or more transducers, one or more sensors and at least one controller are provided. The waveform generators are configured to generate a first and a second drive signal, having two different waveform segments. The system is operable to provide terminal and non-thermal ultrasonic waveforms.

Further relevant prior art is disclosed in documents US 2008/281237 A1, US 2007/239079 A1 and US 2006/184071 A1.

What is needed are new approaches to treating injuries (both chronic and acute) to muscles, tendons, ligaments and cartilage. In addition, new approaches to managing pain are needed. This need is resolved by the subject-matter of the independent claim.

### SUMMARY

The invention is set out in the appended set of claims. Exemplary methods described hereinafter do not form part of the invention.

### DRAWINGS

The present disclosure will become more fully understood from the detailed description and the accompanying drawings, wherein:
Figure 1 illustrates a method of treatment, according to various embodiments;
Figure 2 illustrates a cross sectional view of tissue layers and ultrasound energy directed to a fibrous soft tissue layer, according to various embodiments;
Figure 3 illustrates a cross sectional view of tissue layers and ultrasound energy directed to at least one of muscle and tendon tissues, according to various embodiments;
Figure 4 illustrates a cross sectional view of tissue layers and ultrasound energy directed to at least one of cartilage and ligament tissues, according to various embodiments;
Figure 5 illustrates a cross sectional view of tissue layers and ultrasound energy creating a plurality of lesions in muscle tissue, according to various embodiments;
Figure 6 illustrates a cross sectional view of tissue layers and ultrasound energy creating a plurality of lesions in tendon tissue, according to various embodiments;
Figure 7 illustrates a cross sectional view of tissue layers and a ultrasound probe comprising a movement sensor, according to various embodiments;
Figure 8 illustrates various shapes of lesions, according to various embodiments;
Figure 9 illustrates a treatment system, according to various embodiments;
Figure 10 illustrates a ultrasound probe comprising a transducer and a motion mechanism, according to various embodiments;
Figure 11 illustrates a ultrasound probe comprising a transducer, according to various embodiments;
Figure 12 illustrates a hand held ultrasound probe, according to various embodiments;
Figure 13 illustrates a plurality of exemplary transducer configurations, according to various embodiments;
Figure 14 illustrates methods of treating plantar fascia, according to various embodiments;
Figure 15 illustrates methods of treating plantar fascia strains or partial ruptures, according to various embodiments;
Figure 16 illustrates methods of treating inflamed extensor tendons, according to various embodiments;
Figure 17 illustrates methods of treating an inflamed peroneal tendon, according to various embodiments;
Figure 18 illustrates methods of treating an inflamed tibialis anterior tendon, according to various embodiments;
Figure 19 illustrates methods of treating tears, partial ruptures, or strains in the calf muscle, according to various embodiments;
Figure 20 illustrates methods of treating a strained Achilles tendon, according to various embodiments;
Figure 21 illustrates methods of treating jumper's knee, according to various embodiments;
Figure 22 illustrates methods of treating tears, partial ruptures, or strains in hamstring tendons and hamstring muscles, according to various embodiments;
Figure 23 illustrates methods of treating inflamed portions of the Iliotibial band, according to various embodiments;
Figure 24 illustrates methods of treating tennis elbow, according to various embodiments;
Figure 25 illustrates methods of treating golfer's elbow, according to various embodiments;
Figure 26 illustrates methods of treating a triceps tendon, according to various embodiments;
Figure 27 illustrates methods of treating a biceps tendon, according to various embodiments;
Figure 28 illustrates methods of treating ligaments of the elbow, according to various embodiments;
Figure 29 illustrates methods of treating inflamed tissue near or at a tendon in the forearm, according to various embodiments; and
Figures 30-34 illustrate methods for treating muscles in the shoulder, according to various embodiments.

### DESCRIPTION

The following description is merely exemplary in nature and is in no way intended to limit the various embodiments, their application, or uses. As used herein, the phrase "at least one of A, B, and C" should be construed to mean a logical (A or B or C), using a non-exclusive logical or. As used herein, the phrase "A, B and/or C" should be construed to mean (A, B, and C) or alternatively (A or B or C), using a non-exclusive logical or. It should be understood that steps within a method may be executed in different order without altering the principles of the present disclosure.

The drawings described herein are for illustrative purposes only of selected embodiments and not all possible implementations, and are not intended to limit the scope of any of the various embodiments disclosed herein or any equivalents thereof. It is understood that the drawings are not drawn to scale. For purposes of clarity, the same reference numbers will be used in the drawings to identify similar elements.

The various embodiments may be described herein in terms of various functional components and processing steps. It should be appreciated that such components and steps may be realized by any number of hardware components configured to perform the specified functions. For example, various embodiments may employ various medical treatment devices, visual imaging and display devices, input terminals and the like, which may carry out a variety of functions under the control of one or more control systems or other control devices. In addition, the embodiments may be practiced in any number of medical contexts and that the various embodiments relating to a method and system for acoustic tissue treatment as described herein are merely indicative of exemplary applications for the invention. For example, the principles, features and methods discussed may be applied to any medical application.

According to various embodiments, methods and systems useful for treating sports related injuries are provided herein. The methods and systems provided herein can be noninvasive, for example, no cutting or injecting into the skin is required. Treating an injury to fibrous soft tissue using the methods and systems provided herein minimize recover time and may in some cases eliminate downtime for recovery. Further treating an injury to fibrous soft tissue using the methods and systems provided herein minimize discomfort to a patient having such a procedure.

Various embodiments provide a hand-held extracorporeal system, which emits controlled ultrasound energy into layers of the skin to create a conformal region of elevated temperature in a region of interest that includes an injury location. In one embodiment, a system useful for treating a sports related injury is in a handheld format which may include a rechargeable power supply.

Various embodiments provide a method of treating fibrous soft tissue. The method can include the steps of targeting injured fibrous soft tissue located at or near an injury location; directing therapeutic ultrasound energy to the injured fibrous soft tissue; creating a conformal region of elevated temperature in the injured fibrous soft tissue; and creating at least one thermally induced biological effect in the injured fibrous soft tissue. In various embodiments the thermally induced biological effect can be any one or more than one of, but not limited to, coagulation, increased perfusion, reduction of inflammation, generation of heat shock proteins, and initiation of a healing cascade.

In some embodiments, the method includes the step of imaging the injured fibrous soft tissue. In some embodiments, the method can include driving a medicant into the injured soft fibrous tissue. In one embodiment, the method can include activating the medicant with the therapeutic ultrasound energy. In some embodiments, the method can include peaking inflammation in the injury location and initiating a coagulation cascade in at least a portion of the injured fibrous soft tissue.

In some embodiments, the method can include welding a portion of the injured fibrous soft tissue with the conformal ultrasound energy and repairing a tear in the portion of the injured fibrous soft tissue. In one embodiment, the method includes stimulating collagen growth in a portion of the injured fibrous soft tissue with the conformal ultrasound energy. In some embodiments, the method can further include creating a plurality of micro lesions in a portion of a tendon of the injured fibrous soft tissue and stimulating healing of a plurality of micro tears in the tendon.

In one embodiment, the injured fibrous soft tissue comprises at least a portion of a at least one ligament. In one embodiment, the injured fibrous soft tissue comprises at least a portion of at least one tendon. In one embodiment, the injured soft fibrous soft tissue comprises at least a portion of fascia.

In some embodiments, the fibrous soft tissue comprises at least a portion of an Achilles tendon and the method can include treating at least one of a strain and a partial tear in the Achilles tendon. In some embodiments, the fibrous soft tissue comprises at least a portion of a tendon of extensor of carpi radialis brevis muslien and the method can include treating tennis elbow.

Various embodiments provide a system for treating an injury in fibrous soft tissue. The system can include a hand-held probe and a controller in communication with the probe. In some embodiments, the probe can have a housing. In some embodiments, the housing can contain an ultrasound transducer, a position sensor, a tissue contact sensor, a communication interface, and a rechargeable power supply.

In one embodiment, the ultrasound transducer can be configured to focus a conformal distribution of ultrasound energy into a region of interest comprising a portion of an injury in fibrous soft tissue. In one embodiment, the ultrasound transducer can be configured to image the region of interest comprising the portion of the injury in the fibrous soft tissue.

In some embodiments, a motion mechanism can be coupled to the ultrasound transducer and can be configured to scan the ultrasound transducer in at least one of a linear pattern and a two-dimensional pattern. In some embodiments, the position sensor can be configured to communicate a position of the housing and/or a speed of movement of the housing. In some embodiments, the tissue contact sensor can be configured to communicate a coupling of the transducer to the region of interest.

In some embodiments, the communication interface communicates with the ultrasound transducer, the motion mechanism, the position sensor, and the tissue contact sensor. In some embodiments, the communication interface communicates with the ultrasound transducer, the position sensor, and the tissue contact sensor.

In one embodiment, rechargeable power supply can be one or more batteries. In some embodiments, the rechargeable power supply supplies power to the ultrasound transducer, the motion mechanism, the position sensor, tissue contact sensor, and the communication interface. In some embodiments, the rechargeable power supply supplies power to the ultrasound transducer, the position sensor, tissue contact sensor, and the communication interface.

In some embodiments, the controller communicates with the communication interface and can be configured to control a spatial parameter and a temporal parameter of the ultrasound transducer to emit the conformal distribution of ultrasound energy.

In one embodiment, the controller can receive the position of the housing and/or the speed of movement of the housing, and can control the timing of conformal distribution of ultrasound energy based on the position and/or the speed. In one embodiment, the controller can receive the position of the housing and/or the speed of movement of the housing and can control the scan of the motion mechanism based on the position and/or the speed. In one embodiment, the system can include a second energy source contained in the housing and configured to direct a second energy into the region of interest comprising the injury in a portion of the fibrous soft tissue.

Various embodiments provide a non-invasive method for treating a tear in a portion of fibrous soft tissue. The method can include the steps of identifying a region of interest comprising a tear in a portion of fibrous soft tissue; directing a conformal distribution of ultrasound energy to the region of interest comprising the tear in a portion of fibrous soft tissue; ablating a portion of region of interest comprising the partial rupture in the tear in a portion of fibrous soft tissue; creating a plurality of micro lesions in the region of interest comprising the tear in a portion of fibrous soft tissue; initiating healing of the tear in a portion of fibrous soft tissue; and sparing intervening tissue between the tear in a portion of fibrous soft tissue and a skin surface above the region of interest.

In one embodiment, the method can include welding the tear in a portion of fibrous soft tissue and repairing the tear in a portion of fibrous soft tissue. In one embodiment, the method can include stimulating collagen growth in the portion of fibrous soft tissue. In some embodiments, the method can include creating a three dimensional matrix of micro lesions in the tear in the portion of fibrous soft tissue. In some embodiments, the method can include imaging the region of interest comprising the tear in a portion of fibrous soft tissue. In some embodiments, the method can include increasing blood perfusion to the region of interest comprising the tear in a portion of fibrous soft tissue. In one embodiment, the portion of fibrous soft tissue comprises at least one ligament. In one embodiment, the portion of fibrous soft tissue comprises at least one tendon. In one embodiment, the portion of fibrous soft tissue comprises a portion of fascia.

Various embodiments provide a non-invasive method for treating damaged fibrous soft tissue. The method can include the steps of identifying the damaged fibrous soft tissue; directing a conformal distribution of ultrasound energy to the damaged fibrous soft tissue; ablating a portion of the damaged fibrous soft tissue; creating a plurality of micro lesions in the damaged fibrous soft tissue; initiating healing of the damaged fibrous soft tissue; and sparing intervening tissue between the damaged fibrous soft tissue.

In one embodiment, the method can include stimulating collagen growth in the damaged fibrous soft tissue. In some embodiments, the method can include creating a three dimensional matrix of micro lesions in the damaged fibrous soft tissue. In some embodiments, the method can include imaging the damaged fibrous soft tissue. In some embodiments, the method can include increasing blood perfusion to the damaged fibrous soft tissue. In one embodiment, the damaged fibrous soft tissue comprises at least one ligament. In one embodiment, the damaged fibrous soft tissue comprises at least one tendon. In one embodiment, the damaged fibrous soft tissue comprises a portion of fascia.

Various embodiments provide a method of treating lateral epicondylitis. The method can include the steps of targeting inflamed tissue at a tendon of an extensor carpi radialis brevis musclein; directing therapeutic ultrasound energy to the inflamed tissue; creating a conformal region of elevated temperature in the inflamed tissue; ablating the inflamed tissue; and reducing inflammation in the inflamed tissue.

In some embodiments, the method can include imaging the inflamed tissue. In some embodiments, the method can include driving a medicant into the inflamed tissue. In some embodiments, the method can include activating the medicant with the therapeutic ultrasound energy. In some embodiments, the method can include peaking inflammation in the inflamed tissue and initiating a coagulation cascade in the surrounding tissue. In some embodiments, the method can include welding a portion of the tendon of the extensor carpi radialis brevis musclein with the conformal ultrasound energy. In some embodiments, the method can include stimulating collagen growth in a portion of the tendon of the extensor carpi radialis brevis musclein with the conformal ultrasound energy. In some embodiments, the method can include creating a plurality of micro lesions in a portion of the tendon of the extensor carpi radialis brevis musclein and stimulating healing of a plurality of micro tears in the tendon of the extensor carpi radialis brevis musclein.

Various embodiments provide a system for treating lateral epicondylitis. The system can include a hand held probe and a controller in communication with the hand held probe. In some embodiments, the probe can include a housing, which can contain a dual-function ultrasound transducer, a motion mechanism, a position sensor, a communication interface, and a rechargeable power supply.

In some embodiments, the dual-function ultrasound transducer can be configured to focus a conformal distribution of ultrasound energy into a region of interest comprising a tendon of a extensor carpi radialis brevis musclein and surround subcutaneous tissue. In some embodiments, the dual-function ultrasound transducer can be configured to image a region of interest comprising a tendon of an extensor carpi radialis brevis musclein and the surrounding subcutaneous tissue.

In some embodiments, the motion mechanism is coupled to the dual-function ultrasound transduce. In some embodiments, the motion mechanism can be configured to scan the dual-function ultrasound transducer in a linear pattern or a two-dimensional pattern. In some embodiments, a position sensor can be configured to communicate a position of the housing. In some embodiments, a position sensor can be configured to communicate a speed of movement of the housing.

In some embodiments, the communication interface can be configured for wireless communication. In some embodiments, the communication interface communicates with the dual-function ultrasound transducer, the motion mechanism, and the position sensor. In some embodiments, the rechargeable power supply supplies power to the dual function ultrasound transducer, the motion mechanism, the position sensor, and the communication interface,

In some embodiments, the controller communicates with the communication interface. In some embodiments, the controller controls a spatial parameter and a temporal parameter of the dual-function ultrasound transducer to emit the conformal distribution of ultrasound energy. In some embodiments, the controller receives the position of the housing and the speed of movement of the housing. In some embodiments, the controller controls the timing of conformal distribution of ultrasound energy based on the position and the speed. In some embodiments, the controller controls the scan of the motion mechanism based on the position and the speed. In some embodiments, the controller has a display to display an image of the region of interest.

In some embodiments, the system can include a second energy source contained in the housing. In some embodiments, the second energy source can direct a second energy into the region of interest comprising the tendon of the extensor carpi radialis brevis musclein and the surrounding subcutaneous tissue.

Various embodiments provide a non-invasive method for treating a partial rupture of an Achilles tendon. The method can include the steps of identifying region of interest comprising a tear or partial rupture in an Achilles tendon; directing a conformal distribution of ultrasound energy to region of interest comprising the tear or partial rupture in the Achilles tendon; ablating a portion of region of interest comprising the tear of partial rupture in the Achilles tendon; creating a plurality of micro lesions in region of interest comprising the tear or partial rupture in the Achilles tendon; initiating healing of the tear or partial rupture in the Achilles tendon; and sparing intervening tissue between the tear or partial rupture in the Achilles tendon and a skin surface above the region of interest.

In some embodiments, the method can include welding the tear or partial rupture in the Achilles tendon together. In some embodiments, the method can include stimulating collagen growth in the Achilles tendon. In some embodiments, the method can include creating a three dimensional matrix of micro lesions in the Achilles tendon. In some embodiments, the method can include further comprising imaging the region of interest comprising the tear or partial rupture in the Achilles tendon. In some embodiments, the method can include further comprising peaking inflammation in the surrounding tissue and initiating a coagulation cascade in the Achilles tendon. In some embodiments, the method can include further comprising increasing blood perfusion to the region of interest comprising the tear or partial rupture in an Achilles tendon.

Various embodiments provide a system for treating damaged fibrous soft tissue. The system can include a probe, a controller in communication with the probe, and a power supply. In some embodiments, the probe can include an ultrasound transducer, a motion mechanism, a position sensor, a tissue contact sensor, and a communication interface. In some embodiments, the ultrasound transducer configured to focus a conformal distribution of ultrasound energy into a region of interest comprising damaged fibrous soft tissue. In some embodiments, the ultrasound transducer is configured to image the region of interest comprising the damaged fibrous soft tissue.

In some embodiments, the motion mechanism is coupled to the ultrasound transducer and is configured to scan the ultrasound transducer in at least one of a linear pattern and a two-dimensional pattern. In some embodiments, the position sensor is configured to communicate at least one of a position of the housing and a speed of movement of the housing. In some embodiments, the tissue contact sensor is configured to communicate a coupling of the transducer to the region of interest comprising the damaged fibrous soft tissue. In some embodiments, the communication interface communicates with the ultrasound transducer, the motion mechanism, the position sensor, and the tissue contact sensor.

In various embodiments, the controller communicates with the communication interface and configured to control a spatial parameter and a temporal parameter of the ultrasound transducer to emit the conformal distribution of ultrasound energy. In various embodiments, the power supply configured to supply power to the ultrasound transducer, the motion mechanism, the position sensor, tissue contact sensor, the communication interface, and the controller.

In one embodiment, the controller is configured to receive the at least one of the position of the housing and the speed of movement of the housing, and is configured to control the timing of conformal distribution of ultrasound energy based on at least one of the position and the speed. In one embodiment, the controller is configured to receive the at least one of the position of the housing and the speed of movement of the housing, and is configured to control the scan of the motion mechanism based on at least one of the position and the speed.

In one embodiment, the system can include a display configured to communicate the image of the region of interest comprising the damaged fibrous soft tissue. In some embodiments, the can include a second energy source configured to direct a second energy into the region of interest comprising the damaged fibrous soft tissue. In one embodiment, the second energy source is one of a laser, an intense pulsed light, a light emitting diode, a radiofrequency generator, photon-based energy source, plasma source, a magnetic resonance source, and a mechanical energy source.

Various embodiments provide methods of treating damaged fibrous soft tissue. The method can include targeting a region of interest comprising damaged fibrous soft tissue; directing a biological material to the region of interest; directing ultrasound energy to the region of interest; effecting the biological material with the ultrasound energy; and creating a therapeutic effect with the biological material in the damaged fibrous soft tissue.

In one embodiment, the effecting the biological material with the ultrasound energy is activating the biological material. In one embodiment, the effecting the biological material with the ultrasound energy is increasing adoption of the biological material in the damaged fibrous soft tissue. In one embodiment, the effecting the biological material with the ultrasound energy is potentiating the biological material.

In some embodiments, the method can include creating a conformal region of elevated temperature in the damaged fibrous soft tissue. In some embodiments, the method can include creating a lesion in the damaged fibrous soft tissue and stimulating a wound healing cascade in the region of interest.

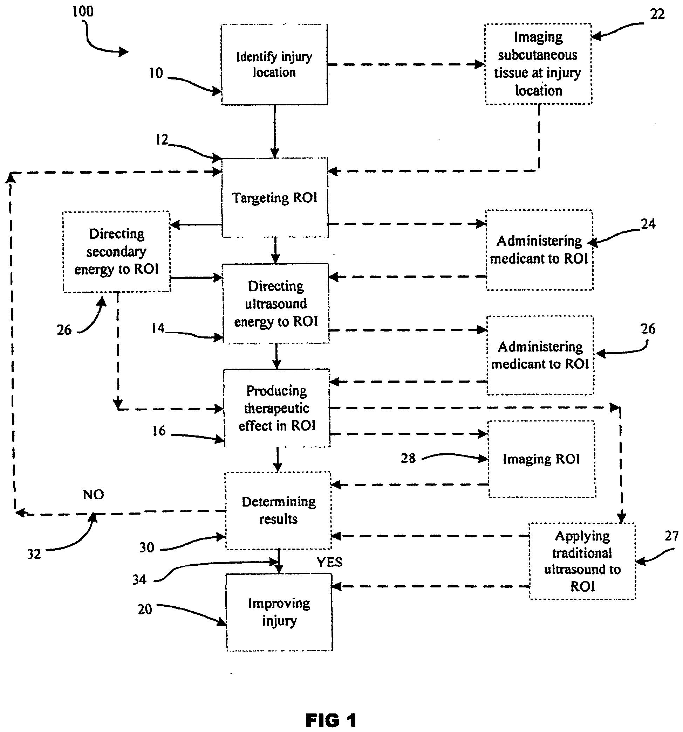

With reference to Figure 1, a method of treatment is illustrated according to various embodiments. Step 10 is identifying the injury location. The injury location maybe anywhere in the body, such as, for example, in any of the following: leg, arm, wrist, hand, ankle, knee, foot, hip, shoulder, back, neck, chest, abdomen, and combinations thereof. Next, Step 12 is targeting a region of interest ("ROI"). The ROI can be located in subcutaneous tissue below the skin surface of the injury location, which can be anywhere in the body, such as, those listed previously.

In various embodiments, the ROI comprises fibrous soft tissue. In some embodiments, the fibrous soft tissue is a muscle and connective tissue layer. In various embodiments, the fibrous soft tissue can comprise any or all of the following tissues: a muscle, a tendon, a ligament, fascia, a sheath, cartilage, and an articular capsule. In various embodiments, a muscle and connective layer is a fibrous connective layer. In various embodiments, the fibrous soft tissue is a fibrous connective tissue layer. In some embodiments, the fibrous soft tissue comprises a tendon. In some embodiments, the fibrous soft tissue comprises a tendon and a sheath. In some embodiments, the fibrous soft tissue comprises a tendon, a sheath, and a portion of muscle connected to the tendon. In some embodiments, the fibrous soft tissue comprises a tendon, fascia, and a muscle connected to the tendon. In some embodiments, the fibrous soft tissue comprises a ligament. In some embodiments, the fibrous soft tissue comprises a ligament and a portion of an articular capsule. In some embodiments, the fibrous soft tissue can include subcutaneous tissue surrounding fibrous connective tissue.

Optionally, step 22 is imaging fibrous soft tissue at the injury location and can be between steps 10 and 12 or can be substantially simultaneous with or be part of step 12. Additionally, imaging may include information from other modalities, such as x-ray, or MRI, which can be imported, linked, or fused. Typically, when the target tissue is a tendon, imaging is used to identify the injury and to direct therapeutic ultrasound energy to precise locations in the ROI without damaging surrounding tissue.

After step 12, step 14 is directing therapeutic ultrasound energy to ROI. The therapeutic ultrasound energy may be focused or unfocused. The therapeutic ultrasound energy can be focused to the fibrous soft tissue layer. The therapeutic ultrasound energy may ablate a portion of the fibrous soft tissue layer. The therapeutic ultrasound energy may coagulate a portion of the fibrous soft tissue layer. The therapeutic ultrasound energy can produce at least one lesion in the fibrous soft tissue layer. The therapeutic ultrasound energy may micro-score a portion of the fibrous soft tissue layer.

The therapeutic ultrasound energy may be streaming. The therapeutic ultrasound energy may be directed to a first depth and then directed to a second depth. The therapeutic ultrasound energy may force a pressure gradient in the fibrous soft tissue layer. The therapeutic ultrasound energy may be cavitation. The therapeutic ultrasound energy may be a first ultrasound energy effect, which comprises an ablative or a hemostatic effect, and a second ultrasound energy effect, which comprises at least one of non-thermal streaming, hydrodynamic, diathermic, and resonance induced tissue effects. Directing therapeutic ultrasound energy to the ROI is a non-invasive technique. As such, the layers above the fibrous soft tissue layer are spared from injury. Such treatment does not require an incision in order to reach the fibrous soft tissue layer to perform treatment for the injury.

In various embodiments, the therapeutic ultrasound energy level for ablating fibrous soft tissue layer is in the range of about 0.1 joules to about 500 joules in order to create an ablative lesion. However, the therapeutic ultrasound energy 108 level can be in the range of from about 0.1 joules to about 100 joules, or from about 1 joules to about 50 joules, or from about 0.1 joules to about 10 joules, or from about 50 joules to about 100 joules, or from about 100 joules to about 500 joules, or from about 50 joules to about 250 joules.

Further, the amount of time therapeutic ultrasound energy is applied at these levels to create a lesion varies in the range from approximately 1 millisecond to several minutes. However, the ranges can be from about 1 millisecond to about 5 minutes, or from about 1 millisecond to about 1 minute, or from about 1 millisecond to about 30 seconds, or from about 1 millisecond to about 10 seconds, or from about 1 millisecond to about 1 second, or from about 1 millisecond to about 0.1 seconds, or about 0.1 seconds to about 10 seconds, or about 0.1 seconds to about 1 second, or from about 1 millisecond to about 200 milliseconds, or from about 1 millisecond to about 0.5 seconds.

The frequency of the ultrasound energy can be in a range from about 0.1 MHz to about 100MHz, or from about 0.1 MHz to about 50MHz, or from about 1MHz to about 50MHz or about 0.1 MHz to about 30 MHz, or from about 10 MHz to about 30 MHz, or from about 0.1MHz to about 20MHz, or from about 1MHz to about 20MHz, or from about 20 MHz to about 30 MHz.

The frequency of the therapeutic ultrasound energy is in the range from about 0.1 MHz to about 50MHz, or about 0.1 MHz to about 30 MHz, or from about 10 MHz to about 30 MHz, or from about 0.1MHz to about 20MHz, or from about 20 MHz to about 30 MHz, or from about 5 MHz to about 15 MHz, or from about 2MHz to about 12 MHz or from about 3MHz to about 7 MHz.

In some embodiments, the ultrasound energy can be emitted to depths at or below a skin surface in a range from about 0 mm to about 150 mm, or from about 0 mm to about 100 mm, or from about 0 mm to about 50 mm, or from about 0 mm to about 30 mm, or from about 0 mm to about 20 mm, or from about 0 mm to about 10 mm, or from about 0 mm to about 5 mm. In some embodiments, the ultrasound energy can be emitted to depths below a skin surface in a range from about 5 mm to about 150 mm, or from about 5 mm to about 100 mm, or from about 5 mm to about 50 mm, or from about 5 mm to about 30 mm, or from about 5 mm to about 20 mm, or from about 5 mm to about 10 mm. In some embodiments, the ultrasound energy can be emitted to depths below a skin surface in a range from about 10 mm to about 150 mm, or from about 10 mm to about 100 mm, or from about 10 mm to about 50 mm, or from about 10 mm to about 30 mm, or from about 10 mm to about 20 mm, or from about 0 mm to about 10 mm.

In some embodiments, the ultrasound energy can be emitted to depths at or below a skin surface in the range from about 20 mm to about 150 mm, or from about 20 mm to about 100 mm, or from about 20 mm to about 50 mm, or from about 20 mm to about 30 mm. In some embodiments, the ultrasound energy can be emitted to depths at or below a skin surface in a range from about 30 mm to about 150 mm, or from about 30 mm to about 100 mm, or from about 30 mm to about 50 mm. In some embodiments, the ultrasound energy can be emitted to depths at or below a skin surface in a range from about 50 mm to about 150 mm, or from about 50 mm to about 100 mm. In some embodiments, the ultrasound energy can be emitted to depths at or below a skin surface in a range from about 20 mm to about 60 mm, or from about 40 mm to about 80 mm, or from about 10 mm to about 40 mm, or from about 5 mm to about 40 mm, or from about 0 mm to about 40 mm, or from about 10 mm to about 30 mm, or from about 5 mm to about 30 mm, or from about 0 mm to about 30 mm.

In various embodiments, a temperature of tissue receiving the ultrasound energy can be in a range from 30°C to about 100°C, or from 43°C to about 60°C, or from 50°C to about 70°C, or from 30°C to about 50°C, or from 43°C to about 100°C, or from 33°C to about 100°C, or from 30°C to about 65°C, or from 33°C to about 70°C, as well as variations thereof. Alternatively, the targeted skin surface and the layers above a target point in the subcutaneous layer are heated to a 10°C to 15°C above the tissue's natural state.

In various embodiments, the ultrasound energy may be emitted at various energy levels, such as for example, the energy levels described herein. Further, the amount of time ultrasound energy is applied at these levels for various time ranges, such as for example, the ranges of time described herein. The frequency of the ultrasound energy is in various frequency ranges, such as for example, the frequency ranges described herein. The ultrasound energy can be emitted to various depths below a targeted skin surface, such as for example, the depths described herein.

Optionally, step 24, which is administering a medicant to the ROI, can be between steps 12 and 14. The medicant can be any chemical or naturally occurring substance that can assist in treating the injury. For example the medicant can be but not limited to a pharmaceutical, a drug, a medication, a nutriceutical, an herb, a vitamin, a cosmetic, an amino acid, a collagen derivative, a holistic mixture, an anti-inflammant, a steroid, a blood vessel dilator or combinations thereof. The medicant can be administered by applying it to the skin above the ROI.

The medicant can be administered by applying it to the skin above ROI. The medicant can be administered to the circulatory system. For example, the medicant can be in the blood stream and can be activated or moved to ROI by the ultrasound energy. The medicant can be administered by injection into or near ROI. Any naturally occurring proteins, stem cells, growth factors and the like can be used as medicant in accordance to various embodiments. A medicant can be mixed in a coupling gel or can be used as a coupling gel.

Step 16 is producing a therapeutic effect in the ROI. A therapeutic effect can be cauterizing and repairing a portion of the fibrous soft tissue layer. A therapeutic effect can be stimulating or increase an amount of heat shock proteins. Such a therapeutic effect can cause white blood cells to promote healing of a portion of the muscle and connective layer in the ROI. A therapeutic effect can be peaking inflammation in a portion of the ROI to decrease pain at the injury location. A therapeutic effect can be creating lesion to restart or increase the wound healing cascade at the injury location. A therapeutic effect can be increasing the blood perfusion to the injury location. Such a therapeutic effect would not require ablative ultrasound energy. A therapeutic effect can be encouraging collagen growth. A therapeutic effect can be relieving pain. A therapeutic effect may increase the "wound healing" response through the liberation of cytokines and may produce reactive changes within the tendon and muscle itself, helping to limit surrounding tissue edema and decrease the inflammatory response to tendon injury.

A therapeutic effect can be synergetic with the medicant administered to ROI in steps 24 and/or 26. A therapeutic effect may be an enhanced delivery of a medicant administered to ROI in steps 24 and/or 26. A therapeutic effect may increase an amount of a medicant administered to ROI in steps 24 and/or 26. A therapeutic effect may be stimulation of a medicant administered to ROI in steps 24 and/or 26. A therapeutic effect may be initiation of a medicant administered to ROI in steps 24 and/or 26. A therapeutic effect may be potentiation of a medicant administered to ROI in steps 24 and/or 26.

A therapeutic effect can be healing an injury to a muscle. A therapeutic effect can be repairing a tendon. A therapeutic effect can be repairing a ligament. A therapeutic effect can be repairing a muscle and a tendon connected to the muscle. Therapeutic effects can be combined.

A therapeutic effect can be produced by a biological effect that initiated or stimulated by the ultrasound energy. A biological effect can be stimulating or increase an amount of heat shock proteins. Such a biological effect can cause white blood cells to promote healing of a portion of the fibrous soft tissue layer. A biological effect can be to restart or increase the wound healing cascade at the injury location. A biological effect can be increasing the blood perfusion to the injury location. A biological effect can be encouraging collagen growth at the injury location. A biological effect may increase the liberation of cytokines and may produce reactive changes within a portion of the fibrous soft tissue layer. A biological effect may by peaking inflammation in a portion of the fibrous soft tissue layer. A biological effect may at least partially shrinking collagen in a portion of the fibrous soft tissue layer. A biological effect may be denaturing of proteins in ROI.

A biological effect may be creating immediate or delayed cell death (apoptosis) in the injury location. A biological effect may be collagen remodeling in the injury location. A biological effect may be the disruption or modification of biochemical cascades in the injury location. A biological effect may be the production of new collagen in the injury location. A biological effect may a stimulation of cell growth in the injury location. A biological effect may be angiogenesis in the injury location. A biological effect may a cell permeability response in the injury location.

A biological effect may be an enhanced delivery of medicants to the injury location. A biological effect may increase an amount of a medicant in the injury location. A biological effect may be stimulation of a medicant in the injury location. A biological effect may be initiation of a medicant in the injury location. A biological effect may be potentiation of a medicant in the injury location.

Optionally, step 26, which is administering medicant to ROI, can be between steps 14 and 16 or can be substantially simultaneous with or be part of step 16. The medicants useful in step 26 are essentially the same as those discussed for step 24.

In various embodiments, ultrasound energy is deposited, which can stimulate a change in at least one of concentration and activity in the injury location of one or more of the following: Adrenomedullin (AM), Autocrine motility factor, Bone morphogenetic proteins (BMPs), Brain-derived neurotrophic factor (BDNF), Epidermal growth factor (EGF), Erythropoietin (EPO), Fibroblast growth factor (FGF), Glial cell line-derived neurotrophic factor (GDNF), Granulocyte colony-stimulating factor (G-CSF), Granulocyte macrophage colony-stimulating factor (GM-CSF), Growth differentiation factor-9 (GDF9), Hepatocyte growth factor (HGF), Hepatoma-derived growth factor (HDGF), Insulin-like growth factor (IGF), Migration-stimulating factor, Myostatin (GDF-8), Nerve growth factor (NGF) and other neurotrophins, Platelet-derived growth factor (PDGF), Thrombopoietin (TPO), Transforming growth factor alpha(TGF-α), Transforming growth factor beta(TGF-β), Tumor necrosis factor-alpha(TNF-α), Vascular endothelial growth factor (VEGF), Wnt Signaling Pathway, placental growth factor (PIGF), [(Foetal Bovine Somatotrophin)] (FBS), IL-1-Cofactor for IL-3 and IL-6, which can activate T cells, IL-2- T-cell growth factor, which can stimulate IL-1 synthesis and can activate B-cells and NK cells, IL-3, which can stimulate production of all non-lymphoid cells, IL-4- Growth factor for activating B cells, resting T cells, and mast cells, IL-5, which can induce differentiation of activated B cells and eosinophils, IL-6, which can stimulate Ig synthesis and growth factor for plasma cells, IL-7 growth factor for pre-B cells, and/or any other growth factor not listed herein, and combinations thereof.

Further, medicants, as described above, can include a drug, a medicine, or a protein, and combinations thereof. Medicants can also include adsorbent chemicals, such as zeolites, and other hemostatic agents are used in sealing severe injuries quickly. Thrombin and fibrin glue are used surgically to treat bleeding and to thrombose aneurysms. Medicants can include Desmopressin is used to improve platelet function by activating arginine vasopressin receptor 1A. Medicants can include coagulation factor concentrates are used to treat hemophilia, to reverse the effects of anticoagulants, and to treat bleeding in patients with impaired coagulation factor synthesis or increased consumption. Prothrombin complex concentrate, cryoprecipitate and fresh frozen plasma are commonly-used coagulation factor products. Recombinant activated human factor VII can be used in the treatment of major bleeding. Medicants can include tranexamic acid and aminocaproic acid, can inhibit fibrinolysis, and lead to a de facto reduced bleeding rate. In addition, medicants can include steroids like the glucocorticoid cortisol.

Optionally, after step 12, step 25, which is directing secondary energy to the ROI, can be substantially simultaneous with or be part of step 16. However, step 25 can be administered at least one of before and after step 16. Step 25 can be alternated with step 16, which can create a pulse of two different energy emissions to the ROI. Secondary energy can be provided by a laser source, or an intense pulsed light source, or a light emitting diode, or a radio frequency, or a plasma source, or a magnetic resonance source, or a mechanical energy source, or any other photon-based energy source. Secondary energy can be provided by any appropriate energy source now known or created in the future. More than one secondary energy source may be used for step 25.

Furthermore, various embodiments provide energy, which may be a first energy and a second energy. For example, a first energy may be followed by a second energy, either immediately or after a delay period. In another example, a first energy and a second energy can be delivered simultaneously. In one embodiment, the first energy and the second energy is ultrasound energy. In some embodiments, the first energy is ultrasound and the second energy is generated by one of a laser, an intense pulsed light, a light emitting diode, a radiofrequency generator, photon-based energy source, plasma source, a magnetic resonance source, or a mechanical energy source, such as for example, pressure, either positive or negative. In other embodiments, energy may be a first energy, a second energy, and a third energy, emitted simultaneously or with a time delay or a combination thereof. In one embodiment, energy may be a first energy, a second energy, a third energy, and an nth energy, emitted simultaneously or with a time delay or a combination thereof. Any of the a first energy, a second energy, a third energy, and a nth nay be generated by at least one of a laser, an intense pulsed light, a light emitting diode, a radiofrequency generator, an acoustic source, photon-based energy source, plasma source, a magnetic resonance source, and/or a mechanical energy source.

Step 20 is improving the injury. Optionally, between steps 16 and 20 is step 30, which is determining results. Between steps 16 and 30 is option step 28, which is imaging the ROI. The images of the ROI from step 28 can be useful for the determining results of step 30. If the results of step 30 are acceptable within the parameters of the treatment then Yes direction 34 is followed to step 20. If the results of step 30 are not acceptable within the parameters of the treatment then No direction 32 is followed back to step 12. After step 16, optionally traditional ultrasound heating can be applied to the ROI in step 27. This application of traditional ultrasound heating to the ROI can be useful in keeping a medicant active or providing heat to support blood perfusion to the ROI after step 16. Further examples and variations of treatment method 100 are discussed herein.

In addition, various different subcutaneous tissues, including for example, muscle and connective layer, may be treated by method 100 to produce different bio-effects, according to some embodiments. Furthermore, any portion of a joint may be treated by method 100 to produce one or more bio-effects, as described herein, in accordance to various embodiments. In order to treat a specific injury location and to achieve a desired bio-effect, therapeutic ultrasound energy may be directed to a specific depth within ROI to reach the targeted subcutaneous tissue, such as, for example, cartilage. For example, if it is desired to cut cartilage by applying therapeutic ultrasound energy at ablative levels, which may be approximately 5 mm to 15 mm below skin surface or at other depths as described herein. An example of ablating cartilage can include a series of lesions ablated into muscle. Besides ablating a portion of cartilage in the joint, other bio-effects may comprise incapacitating, partially incapacitating, severing, rejuvenating, removing, ablating, micro-ablating, shortening, manipulating, or removing tissue either instantly or over time, and combinations thereof.

Depending at least in part upon the desired bio-effect and the subcutaneous tissue being treated, method 100 may be used with an extracorporeal, non-invasive procedure. Also, depending at least in part upon the specific bio-effect and tissue targeted, temperature may increase within ROI may range from approximately 30⁰C to about 60⁰C, or in a range from about 30⁰C to about 100°C, or in other appropriate temperature ranges that are described herein.

In order to treat a specific injury location and to achieve a desired bio-effect, therapeutic ultrasound energy may be directed to a specific depth within ROI to reach the targeted subcutaneous tissue, such as, for example, a fibrous soft tissue layer. For example, if it is desired to cut muscle by applying therapeutic ultrasound energy 120 at ablative levels, which may be approximately 15 mm below skin surface or at other depths as described herein. An example of ablating muscle can include a series of lesions ablated into muscle. Besides ablating fibrous soft tissue layer, other bio-effects may comprise incapacitating, partially incapacitating, severing, rejuvenating, removing, ablating, micro-ablating, shortening, manipulating, or removing tissue either instantly or over time, and combinations thereof.

Depending at least in part upon the desired bio-effect and the subcutaneous tissue being treated, method 100 may be used with an extracorporeal, non-invasive procedure. Also, depending at least in part upon the specific bio-effect and tissue targeted, temperature may increase within ROI may range from approximately 30⁰C to about 60⁰C, or in a range from about 30⁰C to about 100°C, or in other appropriate temperature ranges that are described herein.

In order to treat a specific injury location and to achieve a desired bio-effect, therapeutic ultrasound energy may be directed to a specific depth within ROI to reach the targeted cartilage. Depending at least in part upon the desired bio-effect and the subcutaneous tissue being treated, method 100 may be used with an extracorporeal, non-invasive procedure. Also, depending at least in part upon the specific bio-effect and tissue targeted, temperature may increase within ROI may range from approximately 30⁰C to about 60⁰C, or in a range from about 30⁰C to about 100⁰C, or in other appropriate temperature ranges that are described herein. Also, depending at least in part upon the specific bio-effect and tissue targeted, temperature may increase within ROI may range from approximately 10⁰C to about 15⁰C.

Other bio-effects to target tissue, such as, fibrous soft tissue layer, can include heating, cavitation, streaming, or vibro-accoustic stimulation, and combinations thereof. In various embodiments, therapeutic ultrasound energy is deposited in a matrices of micro-coagulative zones to an already injured tendon or muscle can increase the "wound healing" response through the liberation of cytokines and may produce reactive changes within the tendon and muscle itself, helping to limit surrounding tissue edema and decrease the inflammatory response to tendon injury. In various embodiments, therapeutic ultrasound energy is deposited in a matrices of micro-coagulative zones to an already injured tendon or muscle changes at least one of concentration and activity of inflammatory mediators (TNF-A, IL-1) as well as growth factors (TGF-B1, TGF-B3) at the site of the injure tendon or muscle.

In various embodiments, therapeutic ultrasound energy is deposited in a matrices of micro-coagulative zones to an already injured tendon, muscle, and/or cartilage which can stimulate a change in at least one of concentration and activity of one or more of the following: Adrenomedullin (AM), Autocrine motility factor, Bone morphogenetic proteins (BMPs), Brain-derived neurotrophic factor (BDNF), Epidermal growth factor (EGF), Erythropoietin (EPO), Fibroblast growth factor (FGF), Glial cell line-derived neurotrophic factor (GDNF), Granulocyte colony-stimulating factor (G-CSF), Granulocyte macrophage colony-stimulating factor (GM-CSF), Growth differentiation factor-9 (GDF9), Hepatocyte growth factor (HGF), Hepatoma-derived growth factor (HDGF), Insulin-like growth factor (IGF), Migration-stimulating factor, Myostatin (GDF-8), Nerve growth factor (NGF) and other neurotrophins, Platelet-derived growth factor (PDGF), Thrombopoietin (TPO), Transforming growth factor alpha(TGF-α), Transforming growth factor beta(TGF-β), Tumour necrosis factor-alpha(TNF-α), Vascular endothelial growth factor (VEGF), Wnt Signaling Pathway, placental growth factor (PIGF), [(Foetal Bovine Somatotrophin)] (FBS), IL-1- Cofactor for IL-3 and IL-6, which can activate T cells, IL-2- T-cell growth factor, which can stimulate IL-1 synthesis and can activate B-cells and NK cells, IL-3, which can stimulate production of all non-lymphoid cells, IL-4- Growth factor for activating B cells, resting T cells, and mast cells, IL-5, which can induce differentiation of activated B cells and eosinophils, IL-6, which can stimulate Ig synthesis and growth factor for plasma cells, IL-7 growth factor for pre-B cells, and/or any other growth factor not listed herein, and combinations thereof.

Further, medicants, as described above, can include a drug, a medicine, or a protein, and combinations thereof. Medicants can also include adsorbent chemicals, such as zeolites, and other hemostatic agents are used in sealing severe injuries quickly. Thrombin and fibrin glue are used surgically to treat bleeding and to thrombose aneurysms. Medicants can include Desmopressin is used to improve platelet function by activating arginine vasopressin receptor 1A. Medicants can include coagulation factor concentrates are used to treat hemophilia, to reverse the effects of anticoagulants, and to treat bleeding in patients with impaired coagulation factor synthesis or increased consumption. Prothrombin complex concentrate, cryoprecipitate and fresh frozen plasma are commonly-used coagulation factor products. Recombinant activated human factor VII can be used in the treatment of major bleeding. Medicants can include tranexamic acid and aminocaproic acid, can inhibit fibrinolysis, and lead to a de facto reduced bleeding rate. In addition, medicant can include steroids, (anabolic steroids and/or costisol steroids), for example glucocorticoid cortisol or prednisone. Medicant can include can include compounds as alpha lipoic acid, DMAE, vitamin C ester, tocotrienols, and phospholipids.

Medicant can be a pharmaceutical compound such as for example, cortisone, Etanercept, Abatacept, Adalimumab, or Infliximab. Medicant can include platelet-rich plasma (PRP), mesenchymal stem cells, or growth factors. For example, PRP is typically a fraction of blood that has been centrifuged. The PRP is then used for stimulating healing of the injury. The PRP typically contains thrombocytes (platelets) and cytokines (growth factors). The PRP may also contain thrombin and may contain fibenogen, which when combined can form fibrin glue. Medicant can be a prothrombin complex concentrate, cryoprecipitate and fresh frozen plasma, which are commonly-used coagulation factor products. Medicant can be a recombinant activated human factor VII, which can be used in the treatment of major bleeding. Medicant can include tranexamic acid and aminocaproic acid, can inhibit fibrinolysis, and lead to a de facto reduced bleeding rate. In some embodiments, medicant can be Botox. In addition, medicants can include steroids like the glucocorticoid cortisol.

According to various embodiments of method 100, ultrasound probe is coupled directly to ROI, as opposed to skin surface 104, to treat targeted tissue. For example, ultrasound probe can be integrated to or attached to a tool, such as, for example, an arthroscopic tool, laparoscopic tool, or an endoscopic tool that may be inserted into a patient's body with minimal invasiveness.

According to various embodiments of method 100, ultrasound probe is coupled directly to ROI, as opposed to skin surface, to treat targeted tissue. For example, ultrasound probe can be integrated to or attached to a tool, such as, for example, an arthroscopic tool, laparoscopic tool, or an endoscopic tool that may be inserted into a patient's body with minimal invasiveness.

In various embodiments, method 100 can treat either recent or older injuries, or combinations thereof. Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process. In various embodiments, method 100 can treat chronic inflammation. In various embodiments, method 100 can treat acute inflammation. In some embodiments, method 100 can treat a combination of acute and chronic inflammation.

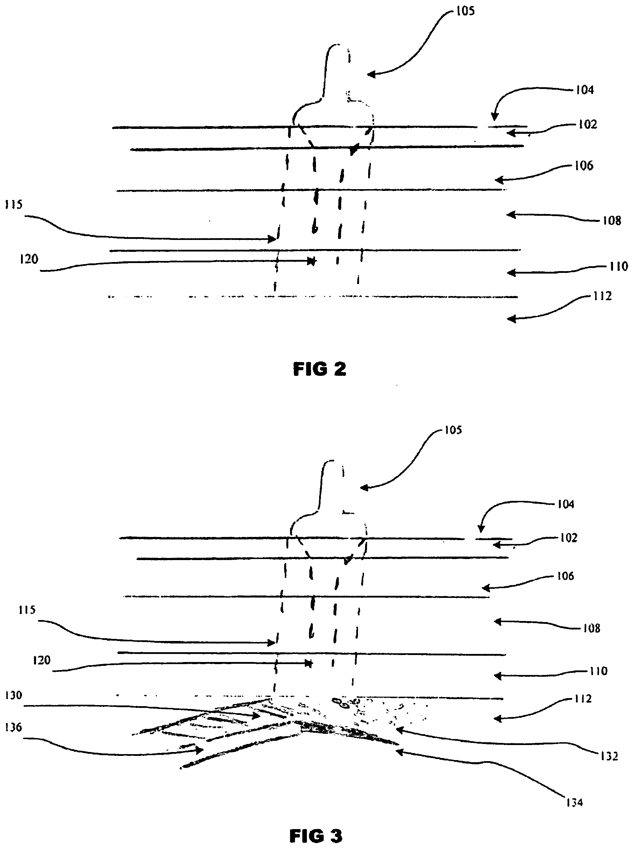

Now moving to Figure 2, a cross sectional view of tissue layers and ultrasound energy directed to a fibrous soft tissue layer, according to various embodiments, is illustrated. In various embodiments, ultrasound energy 120 creates a conformal region of elevated temperature. In some embodiments, conformal region of elevated temperature is a conformal energy deposition, which increases the temperature in a conformal region of tissue in ROI 115 by about 5°C to 65°C above the internal body temperature or higher. In some embodiments, conformal region of elevated temperature is a conformal energy deposition, which is placed at a selected depth in the tissue in ROI 115 and has a defined shape and volume. In some embodiments, conformal region of elevated temperature is a shaped conformal distribution of elevated temperature in ROI 115, which can be created through adjustment of the strength, depth, and type of focusing, energy levels and timing cadence.

In various embodiment, ultrasound probe 105 is configured with the ability to controllably produce conformal distribution of elevated temperature in soft tissue within ROI 115 through precise spatial and temporal control of acoustic energy deposition, i.e., control of ultrasound probe 105 is confined within selected time and space parameters, with such control being independent of the tissue. The ultrasound energy 120 can be controlled to produce a conformal distribution of elevated temperature in soft tissue within ROI 115 using spatial parameters. The ultrasound energy 120 can be controlled to produce conformal distribution of elevated temperature in soft tissue within ROI 115 using temporal parameters. The ultrasound energy 120 can be controlled to produce a conformal distribution of elevated temperature in soft tissue within ROI 115 using a combination of spatial parameters and temporal parameters. In some embodiments, a conformal distribution of elevated temperature in soft tissue within ROI 115 is conformal region of elevated temperature in ROI 115.

In various embodiments, conformal region of elevated temperature can create a lesion in ROI 115. In various embodiments, conformal region of elevated temperature can initiate thermal injury in a portion of ROI 115. In various embodiments, conformal region of elevated temperature can initiate or stimulate coagulation in a portion of ROI 115. In various embodiments, conformal region of elevated temperature can be one of a series of micro scoring in ROI 115. In various embodiments, conformal region of elevated temperature can with a first ultrasound energy deposition and a second energy deposition. In one embodiment, second energy deposition is ultrasound energy. In some embodiments, second energy is any one of second energy that may be used for method 100, as discussed herein.

In various embodiments, conformal region of elevated temperature can stimulate and/or initiate a therapeutic effect. In various embodiments, conformal region of elevated temperature can stimulate and/or initiate a biological effect. In various embodiments, conformal region of elevated temperature can denature tissue in ROI 115. In various embodiments, conformal region of elevated temperature can drive a medicant into ROI 115. In various embodiments, conformal region of elevated temperature can activate a medicant in ROI 115. In various embodiments, conformal region of elevated temperature can create immediate or delayed cell death (apoptosis) in the ROI. In various embodiments, conformal region of elevated temperature can create one or more ablation zones in ROI 115. In various embodiments, conformal region of elevated temperature can increase blood perfusion in ROI 115.

In one embodiment, conformal region of elevated temperature can be created by heating a portion of ROI 115 with ultrasound energy 120. In one embodiment, conformal region of elevated temperature can be created by cavitation in ROI 115, which is initiated by ultrasound energy 120. In one embodiment, conformal region of elevated temperature can be created by streaming ultrasound energy 120 into ROI 115. In one embodiment, conformal region of elevated temperature can be created by vibro-accoustic stimulation in ROI 115, which is initiated by ultrasound energy 120. In one embodiment, conformal region of elevated temperature can be created by a combination of two or more of heating, cavitation, streaming, or vibro-accoustic stimulation.

In some embodiments, conformal region of elevated temperature can be a shaped lesion, which can be created through adjustment of the strength, depth, and type of focusing, energy levels and timing cadence. For example, focused ultrasound energy 120 can be used to create precise arrays of microscopic thermal ablation zones. Ultrasound energy 120 can produce an array of ablation zones deep into the layers of the soft tissue. Detection of changes in the reflection of ultrasound energy can be used for feedback control to detect a desired effect on the tissue and used to control the exposure intensity, time, and/or position. In various embodiments, ultrasound probe 105 is configured with the ability to controllably produce conformal region of elevated temperature in soft tissue within ROI 115 through precise spatial and temporal control of acoustic energy deposition, i.e., control of ultrasound probe 105 is confined within selected time and space parameters, with such control being independent of the tissue.

In accordance with various embodiments, ultrasound probe 105 can be configured for spatial control of ultrasound energy 120 by controlling the manner of distribution of the ultrasound energy 120 to create conformal region of elevated temperature. For example, spatial control may be realized through selection of the type of one or more spatial parameters of the transducer configurations insonifying ROI 115, selection of the placement and location of ultrasound probe 105 for delivery of ultrasound energy 120 relative to ROI 115 e.g., ultrasound probe 105 being configured for scanning over part or whole of ROI 115 to produce a contiguous conformal region of elevated temperature having a particular orientation or otherwise change in distance from ROI 115, and/or control of other environment parameters, e.g., the temperature at the acoustic coupling interface can be controlled, and/or the coupling of ultrasound probe 105 to tissue. Other spatial control can include but are not limited to geometry configuration of ultrasound probe 105 or transducer assembly, lens, variable focusing devices, variable focusing lens, stand-offs, movement of ultrasound probe, in any of six degrees of motion, transducer backing, matching layers, number of transduction elements in transducer, number of electrodes, or combinations thereof.

In various embodiments, ultrasound probe 105 can also be configured for temporal control of ultrasound energy 120 by controlling the timing of the distribution of the ultrasound energy 120 to create conformal region of elevated temperature. For example, temporal control may be realized through adjustment and optimization of one or more temporal parameters, such as for example, drive amplitude levels, frequency, waveform selections, e.g., the types of pulses, bursts or continuous waveforms, and timing sequences and other energy drive characteristics to control thermal ablation of tissue. Other temporal parameters can include but are not limited to full power burst of energy, shape of burst, timing of energy bursts, such as, pulse rate duration, continuous, delays, etc., change of frequency of burst, burst amplitude, phase, apodization, energy level, or combinations thereof.

The spatial and/or temporal control can also be facilitated through open-loop and closed-loop feedback arrangements, such as through the monitoring of various spatial and temporal characteristics. As a result, control of acoustical energy within six degrees of freedom, e.g., spatially within the X, Y and Z domain, as well as the axis of rotation within the XY, YZ and XZ domains, can be suitably achieved to generate conformal region of elevated temperature of variable shape, size and orientation. For example, through such spatial and/or temporal control, ultrasound probe 105 can enable the regions of thermal injury to possess arbitrary shape and size and allow the tissue to be destroyed (ablated) in a controlled manner.

The tissue layers illustrated in Figure 2 are skin surface 104, epidermal layer 102, dermis layer 106, fat layer 108, SMAS layer 110, and fibrous soft tissue layer 112. Ultrasound probe 105 emits therapeutic ultrasound energy 120 in ROI 115. In various embodiments, ultrasound probe 105 is capable of emitting therapeutic ultrasound energy 120 at variable depths in ROI 115, such as, for example, the depths described herein. Ultrasound probe 105 is capable of emitting therapeutic ultrasound energy as a single frequency, variable frequencies, or a plurality of frequencies, such as, for example, the frequency ranges described herein. Ultrasound probe 105 is capable of emitting therapeutic ultrasound energy 120 for variable time periods or to pulse the emission over time, such as, for example, those time intervals described herein. Ultrasound probe 105 is capable of providing various energy levels of therapeutic ultrasound energy, such as, for example, the energy levels described herein. Ultrasound probe 105 may be individual hand-held device, or may be part of a treatment system. The ultrasound probe 105 can provide both therapeutic ultrasound energy and imaging ultrasound energy. However, ultrasound probe 105 may provide only therapeutic ultrasound energy. Ultrasound probe 105 may comprise a therapeutic transducer and a separate imaging transducer. Ultrasound probe 105 may comprise a transducer or a transducer array capable of both therapeutic and imaging applications. According an alternative embodiment, ultrasound probe 105 is coupled directly to one of the tissue layers, as opposed to skin surface 104 to treat the tissue layer. For example, ultrasound probe can be integrated to or attached to a tool, such as, for example, an arthroscopic tool, laparoscopic tool, or an endoscopic tool that may be inserted into a patient's body with minimal invasiveness.

In various embodiments, ultrasound probe 105 may be used for method 100. In various embodiments, method 100 can be implemented using any or all of the elements illustrated in Figure 2. As will be appreciated by those skilled in the art, at least a portion of method 100 or a variation of method 100 can be implemented using any or all of the elements illustrated in Figure 2.

With reference to Figure 3, a cross sectional view of tissue layers and ultrasound energy directed to at least one of muscle 130 and tendon 134, according to various embodiments, is illustrated. The tissue layers illustrated are skin surface 104, epidermal layer 102, dermis layer 106, fat layer 108, SMAS layer 110, tendon 134, muscle 130, and fat 132. In some embodiments, ROI 115 comprises at least one of muscle 130 and tendon 134. In some embodiments, ROI 115 can comprise skin surface 104, epidermal layer 102, dermis layer 106, fat layer 108, SMAS layer 110, and fibrous soft tissue layer 112, which comprises tendon 134, muscle 130, and fat 132. In some embodiments, ultrasound probe 105 images at least a portion of one of skin surface 104, epidermal layer 102, dermis layer 106, fat layer 108, SMAS layer 110, and fibrous soft tissue layer 112, which comprises tendon 134, muscle 130, and fat 132. In one embodiment, ultrasound probe 105 images at least one muscle 130 and tendon 134. Ultrasound probe 105 emits therapeutic ultrasound energy 120 to at least one of muscle 130 and tendon 134.

As well known to those skilled in the art, tendon 134 attaches muscle 130 to bone 136. In various embodiments, therapeutic ultrasound energy 120 treats at least one of muscle 130 and tendon 134. In one embodiment, therapeutic ultrasound energy 120 ablates a portion of at least one a muscle 130 and tendon 134 creating a lesion. In one embodiment therapeutic ultrasound energy 120 coagulates a portion of at least one of muscle 130 and tendon 134. According an alternative embodiment, ultrasound probe 105 is coupled directly to a portion of at least one of muscle 130 and tendon 134, as opposed to skin surface 104, to treat the a portion of at least one of muscle 130 and tendon 134. For example, ultrasound probe can be integrated to or attached to a tool, such as, for example, an arthroscopic tool, laparoscopic tool, or an endoscopic tool that may be inserted into a patient's body with minimal invasiveness. In various embodiments, ultrasound probe 105 may be used for method 100. In various embodiments, method 100 can be implemented using any or all of the elements illustrated in Figure 3. As will be appreciated by those skilled in the art, at least a portion of method 100 or a variation of method 100 can be implemented using any or all of the elements illustrated in Figure 3.

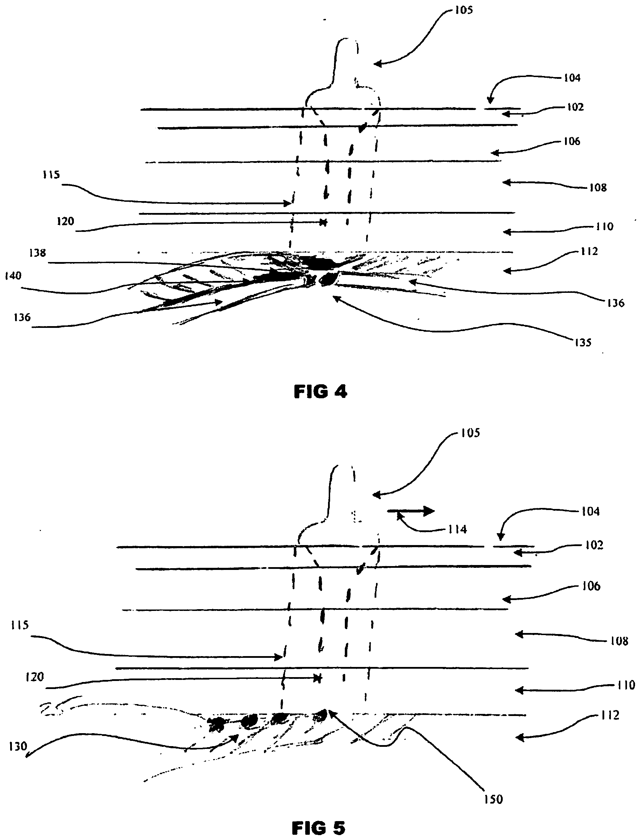

In Figure 4, a cross sectional view of tissue layers and ultrasound energy directed to at least one of cartilage 140 and ligament 138, according to various embodiments, is illustrated. The tissue layers illustrated are skin surface 104, epidermal layer 102, dermis layer 106, fat layer 108, SMAS layer 110, and fibrous soft tissue layer 112, which comprises cartilage 140 and ligament 138. As well known to those skilled in the art, joint 135 can comprise ligament 138, cartilage 140, and bone 136. In some embodiments, ROI 115 comprises at least one of cartilage 140 and ligament 138. In some embodiments, ROI 115 can comprise at least a portion of joint 135. ROI 115 can comprise any or all of the following: skin surface 104, epidermal layer 102, dermis layer 106, fat layer 108, SMAS layer 110, and muscle and connective tissue 112, which comprises ligament 138 and cartilage 140. In some embodiments, ultrasound probe 105 can image at least a portion of one of skin surface 104, epidermal layer 102, dermis layer 106, fat layer 108, SMAS layer 110, ligament 138 and cartilage 140. Ultrasound probe 105 emits therapeutic ultrasound energy 120 to ligament 138. In various embodiments, therapeutic ultrasound energy 120 treats ligament 138. In various embodiments, therapeutic ultrasound energy 120 treats at least a portion of joint 135. According an alternative embodiment, ultrasound probe 105 is coupled directly to a portion of joint 135, as opposed to skin surface 104, to treat the a portion of joint 135. For example, ultrasound probe can be integrated to or attached to a tool, such as, for example, an arthroscopic tool, laparoscopic tool, or an endoscopic tool that may be inserted into a patient's body with minimal invasiveness. In various embodiments, ultrasound probe 105 may be used for method 100. In various embodiments, method 100 can be implemented using any or all of the elements illustrated in Figure 4. As will be appreciated by those skilled in the art, at least a portion of method 100 or a variation of method 100 can be implemented using any or all of the elements illustrated in Figure 4.

In one embodiment, therapeutic ultrasound energy 120 ablates a portion of a ligament 138 creating a lesion. In one embodiment, therapeutic ultrasound energy 120 ablates a portion of joint 135 creating a lesion. In one embodiment therapeutic ultrasound energy coagulates a portion of ligament 138. In one embodiment therapeutic ultrasound energy 120 coagulates a portion of joint 135.

Referring to Figure 5, a cross sectional view of tissue layers and ultrasound energy creating a plurality of lesions in muscle tissue, according to various embodiments, is illustrated. The tissue layers illustrated are skin surface 104, epidermal layer 102, dermis layer 106, fat layer 108, SMAS layer 110, and muscle 130. In some embodiments, ROI 115 comprises a portion of muscle 130. In some embodiments, ROI 115 can comprise skin surface 104, epidermal layer 102, dermis layer 106, fat layer 108, SMAS layer 110, and fibrous soft tissue layer 112, which comprises at least a portion of muscle 130. In some embodiments, ultrasound probe 105 images at least a portion of one of skin surface 104, epidermal layer 102, dermis layer 106, fat layer 108, SMAS layer 110, and fibrous soft tissue layer 112, which comprises at least a portion of muscle 130. In one embodiment, ultrasound probe 105 images at least a portion of muscle 130. Ultrasound probe 105 emits therapeutic ultrasound energy 120 to at least a portion of muscle 130. In various embodiments, therapeutic ultrasound energy 120 treats a portion of muscle 130. In various embodiments, ultrasound probe 105 may be used for method 100. In various embodiments, method 100 can be implemented using any or all of the elements illustrated in Figure 5. As will be appreciated by those skilled in the art, at least a portion of method 100 or a variation of method 100 can be implemented using any or all of the elements illustrated in Figure 5.

In some embodiments, ultrasound probe 105 can be moved in at least one direction 114 to provide a plurality of lesions 25 in muscle 130. In various embodiments, a plurality of lesions 25 can be placed in a pattern in a portion of muscle 130, such as, for example, a 1-D pattern, a 2-D pattern, a 3-D pattern, or combinations thereof. In one embodiment, therapeutic ultrasound energy 120 ablates a portion muscle 130 creating lesion 25. In one embodiment, therapeutic ultrasound energy 120 ablates a portion muscle 130 creating lesion 25. In one embodiment, therapeutic ultrasound energy 120 coagulates a portion of muscle 130.

Therapeutic ultrasound energy 120 creates ablation zone 150 in a tissue layer, at which a temperature of tissue is raised to at least 43⁰C, or is raised to a temperature in the range form about 43⁰C to about 100°C, or from about 50⁰C to about 90⁰C, or from about 55⁰C to about 75⁰C, or from about 50⁰C to about 65⁰C, or from about 60⁰C to about 68⁰C.

In some embodiments, ultrasound probe 105 can be moved in at least one direction 114 to provide a plurality of lesions 25 in a tissue layer. In various embodiments, a plurality of lesions 25 can be placed in a pattern in at least one tissue layer, such as, for example, a 1-D pattern, a 2-D pattern, a 3-D pattern, or combinations thereof. In one embodiment, ultrasound probe 105 comprises a single transducer element and while emitting therapeutic ultrasound energy 120 in a pulsed matter, is moved in a linear motion along skin surface 104 to create a 1-D pattern of a plurality of lesions 25 in at least one tissue layer. In one embodiment, ultrasound probe 105 comprises a linear array of transducers and while emitting therapeutic ultrasound energy 120 in a pulsed matter, is moved along the linear vector of the array on skin surface 104 to create a 1-D pattern of a plurality of lesions 25 in at least one tissue layer.

In one embodiment, ultrasound probe 105 comprises a linear array of transducers and while emitting therapeutic ultrasound energy 120 in a pulsed matter, is moved along the non-linear vector of the array on skin surface 104 to create a 2-D pattern of a plurality of lesions 25 in at least one tissue layer. In one embodiment, ultrasound probe 105 comprises an array of transducers and while emitting therapeutic ultrasound energy 120 in a pulsed matter, is moved along skin surface 104 to create a 2-D pattern of a plurality of lesions 25 in at least one tissue layer.

In one embodiment, ultrasound probe 105 comprises an array of transducers, wherein the array comprises a first portion focusing to a first depth and a second portion focusing to a second depth, and while emitting therapeutic ultrasound energy 120 in a pulsed matter, is moved along skin surface 104 to create a 3-D pattern of a plurality of lesions 25 in at least one tissue layer. In one embodiments, ultrasound probe 105 comprises at least two arrays of transducers, wherein a first array focusing to a first depth and a second array focusing to a second depth, and while each of the arrays emitting therapeutic ultrasound energy 120 in a pulsed matter, is moved along skin surface 104 to create a 3-D pattern of a plurality of lesions 25 in at least one tissue layer. In one embodiment, ultrasound probe 105 comprises a linear array of transducers and while emitting therapeutic ultrasound energy 120 in a pulsed matter, is moved along the non-linear vector of the array on skin surface 104 focused to a first depth then moved in the same direction along skin surface focused at a second depth to create a 3-D pattern of a plurality of lesions 25 in at least one tissue layer. In one embodiment, ultrasound probe 105 comprises an array of transducers and while emitting therapeutic ultrasound energy 120 in a pulsed matter, is moved along skin surface 104 focused to a first depth then moved in the same direction along skin surface focused at a second depth to create a 3-D pattern of a plurality of lesions 25 in at least one tissue layer.

In various embodiments, methods of building muscle are provided. The method can include targeting the muscle 130 to be strengthened, directing therapeutic ultrasound energy to the muscle 130, creating a pattern of a plurality of lesions 25, allowing the muscle to heal, thereby strengthening the muscle 130. In addition, such methods can useful for building muscle 130 mass. Still further, such methods can be useful for treating stroke victims.

A tendon is a tough yet flexible band of fibrous connective tissue that usually connects muscle to bone, it transmits the force of the muscle contraction to the bone which enables movement. Normal healthy tendons are composed of parallel arrays of collagen fibers closely packed together. The fibers are mostly collagen type I, however, both collagen type III and V may be present. Collagen molecules are produced by tenocytes and aggregate end-to-end and side-to side to produce collagen fibrils, organized fibril bundles form fibers, groups of fibers form macroaggregates, groups of macroaggregates bounded by endotendon form fascicles and groups of fascicles bounded by epitendon and peritendon form the tendon organ.

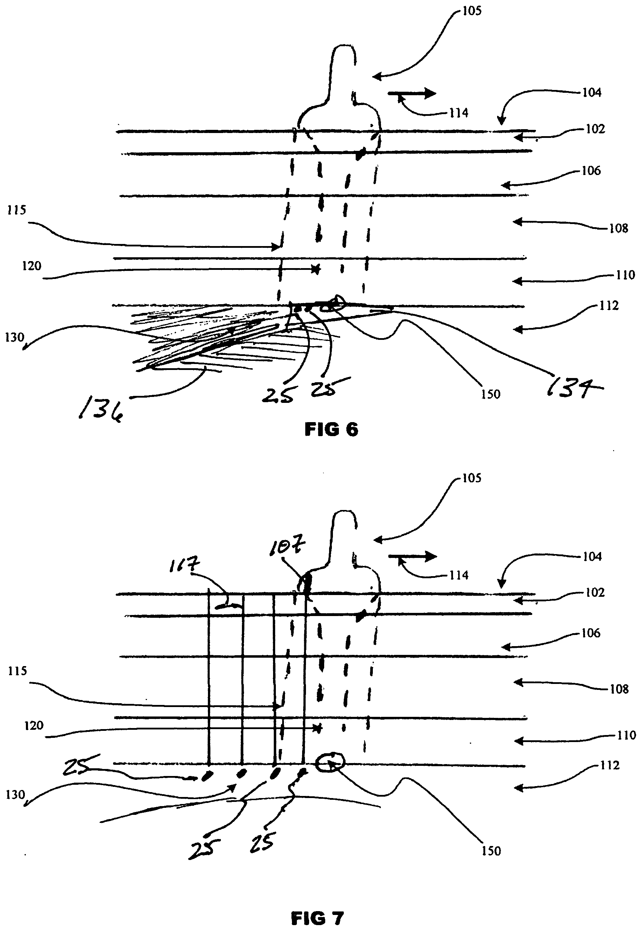

Now with reference to Figure 6, a cross sectional view of tissue layers and ultrasound energy creating a plurality of lesions in tendon tissue, according to various embodiments is illustrated. The tissue layers illustrated are skin surface 104, epidermal layer 102, dermis layer 106, fat layer 108, SMAS layer 110, and tendon 134. In some embodiments, ROI 115 comprises a portion of tendon 134. In some embodiments, ROI 115 can comprise skin surface 104, epidermal layer 102, dermis layer 106, fat layer 108, SMAS layer 110, and fibrous soft tissue layer 112, which comprises at least a portion of tendon 134. In some embodiments, ultrasound probe 105 images at least a portion of one of skin surface 104, epidermal layer 102, dermis layer 106, fat layer 108, SMAS layer 110, and fibrous soft tissue layer 112, which comprises at least a portion of tendon 134. In one embodiment, ultrasound probe 105 images at least a portion of tendon 134. Ultrasound probe 105 emits therapeutic ultrasound energy 120 to at least a portion of tendon 134. In various embodiments, therapeutic ultrasound energy 120 treats a portion of tendon 134. In various embodiments, ultrasound probe 105 may be used for method 100. In various embodiments, method 100 can be implemented using any or all of the elements illustrated in Figure 6. As will be appreciated by those skilled in the art, at least a portion of method 100 or a variation of method 100 can be implemented using any or all of the elements illustrated in Figure 6.

In some embodiments, ultrasound probe 105 can be moved in at least one direction 114 to provide a plurality of lesions 25 in tendon 134. In various embodiments, a plurality of lesions 25 can be placed in a pattern in portion of tendon 134, such as, for example, a 1-D pattern, a 2-D pattern, a 3-D pattern, or combinations thereof. A variety of exemplary embodiments of creating lesions patterns are described herein. In one embodiment, therapeutic ultrasound energy 120 ablates a portion tendon 134 creating lesion 25. In one embodiment, therapeutic ultrasound energy 120 ablates a portion tendon 134 creating lesion 25. Therapeutic ultrasound energy 120 creates ablation zone 150 in a portion of tendon 134. Various temperature ranges of ablation zone 150 are described herein. In one embodiment, therapeutic ultrasound energy 120 coagulates a portion of tendon 134. As will be appreciated by those skilled in the art, a treatment plan can include a plurality of lesions 25 in a portion of muscle 130 and tendon 134.

Moving to Figure 7, a cross sectional view of tissue layers and ultrasound probe comprising a movement sensor, according to various embodiments, is illustrated. The tissue layers illustrated are skin surface 104, epidermal layer 102, dermis layer 106, fat layer 108, SMAS layer 110, and fibrous soft tissue layer 112. In some embodiments, ROI 115 comprises a portion of fibrous soft tissue layer 112. In some embodiments, ROI 115 can comprise skin surface 104, epidermal layer 102, dermis layer 106, fat layer 108, SMAS layer 110, and fibrous soft tissue layer 112.

In some embodiments, ultrasound probe 105 images at least a portion of one of skin surface 104, epidermal layer 102, dermis layer 106, fat layer 108, SMAS layer 110, and fibrous soft tissue layer 112. Ultrasound probe 105 emits therapeutic ultrasound energy 120 to at least a portion of a fibrous soft tissue layer 112. In various embodiments, therapeutic ultrasound energy 120 treats a portion of fibrous soft tissue layer 112. In various embodiments, ultrasound probe 105 may be used for method 100. In various embodiments, method 100 can be implemented using any or all of the elements illustrated in Figure 7. As will be appreciated by those skilled in the art, at least a portion of method 100 or a variation of method 100 can be implemented using any or all of the elements illustrated in Figure 7.

In some embodiments, ultrasound probe 105 can be moved in at least one direction 114 to provide a plurality of lesions 25 in fibrous soft tissue layer 112. In various embodiments, a plurality of lesions 25 can be placed in a pattern in a portion of fibrous soft tissue layer 112, such as, for example, a 1-D pattern, a 2-D pattern, a 3-D pattern, or combinations thereof. A variety of exemplary embodiments of creating lesions patterns are described herein. However, in various embodiments, ultrasound probe comprises position sensor 107. Position sensor 107 can be integrated into ultrasound probe 105 or attached to ultrasound probe 105. In one embodiment, position sensor 107 is a motion sensor measuring movement of ultrasound probe 105. Such a motion sensor can calculate distance traveled along skin surface 104. Such a motion sensor may determine a speed of movement of ultrasound probe 105 along skin surface 104 and determine if the speed is accurate for treatment. For example if the speed is too fast, motion sensor can signal an indicator to slow the speed and/or can signal transducer to stop emitting therapeutic ultrasound energy 120.

In various embodiments, position sensor 107 comprises a visual element such as a camera or video capture device. In such embodiments, skin surface 104 can be geotagged. Features on the skin surface, such as, for example, a scar, a nipple, a belly button, a mole, an ankle, a knee cap, a hip bone, a mark, a tattoo, or combinations thereof and the like, may be geotagged using position sensor 107. A geotagged feature may be useful for treatment. A geotagged feature may be useful for setting parameters for treatment. A geotagged feature may be useful for determining progress or success of treatment. A geotagged feature may be useful to position ultrasound probe for a second treatment of injury location. A geotagged feature can be stored with other treatment parameters and/or treatment results.

In various embodiments, position sensor 107 can include a laser position sensor. For example, position sensor 107 can track position like a computer mouse that uses a laser sensor as opposed to an older version of a mouse with a roller ball. Position sensor 107 can communicate to a display to track a position of ultrasound probe 105, such as, for example, overlaid on an image of ROI 115, overlaid on an image of skin surface 104, as referenced to geotagged features, as reference to injury location, as referenced to a prior treatment, and combinations thereof. In one a treatment plan can include a movement pattern of ultrasound probe 105. Such a movement pattern can be displayed and the position sensor 107 can track a position of ultrasound probe 105 during treatment as compared to the movement pattern. Tracking ultrasound probe 105 with position sensor and comparing the tracked movement to a predetermined movement may be useful as a training tool. In one embodiment, laser position sensor can geotag a feature on skin surface 104. In various embodiments, position sensor 107 may determine a distance 117 between pulses of therapeutic ultrasound energy 120 to create a plurality of lesions 25 which are evenly space. As ultrasound probe 105 is moved in direction 114, position sensor 107 determines distance 117, regardless of a speed that ultrasound probe 105 is move, at which a pulse of therapeutic ultrasound energy 120 is to be emitted in to ROI 115.

Position sensor 107 may be located behind the transducer element, in front of the transducer element, or integrated into the transducer element. Ultrasound probe 105 may comprise more than one position sensor 107, such as, for example, a laser position sensor and a motion sensor, or a laser position sensor and a visual device, or a motion sensor and a visual device, or a laser position sensor, a motion sensor, and a visual device. Additional embodiments of position sensor 107 may be found in US Patent No. 7,142,905, entitled "Visual Imaging System for Ultrasonic Probe" issued November 28, 2006, and US Patent No. 6,540,679, entitled "Visual Imaging System for Ultrasonic Probe" issued April 1, 2003.

In various embodiments, ultrasound probe 105 can comprise a tissue contact sensor. In one embodiment, tissue contact sensor communicates whether ultrasound probe 105 is coupled to the ROI 115. The tissue contact sensor may measure a capacity of a skin surface 104 above the ROI 115 and communicate a difference between the capacity of the contact to the skin surface 104 and the capacity of air. In one embodiment, the tissue contact sensor is initiated or turned on by pressing ultrasound probe against skin surface 104.

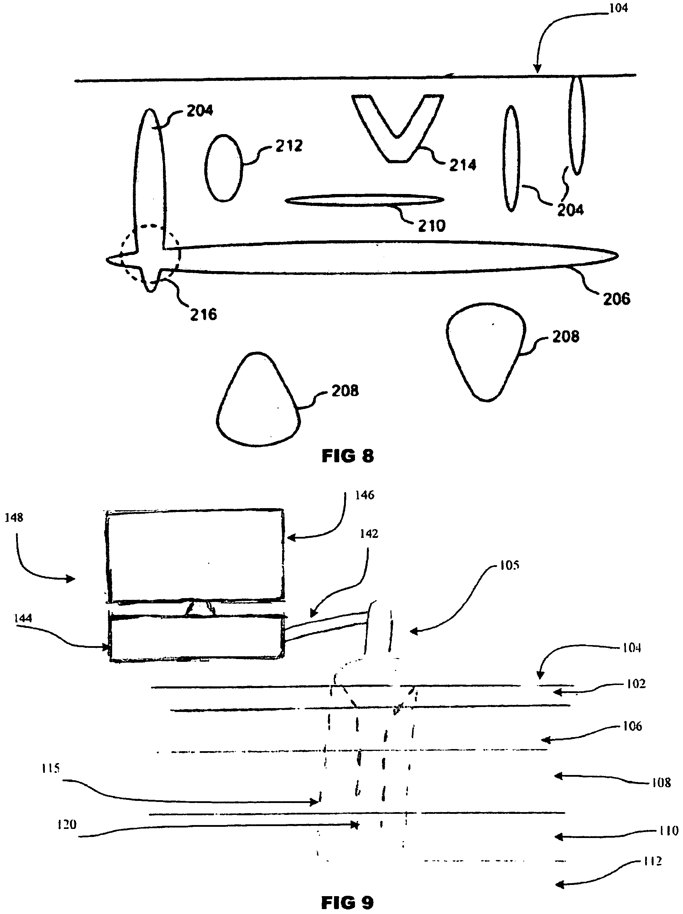

Referring to Figure 8 various shapes of lesions, according to various embodiments, are illustrated. In various embodiment, ultrasound probe 105 is configured with the ability to controllably produce conformal lesions 25 of thermal injury in fibrous soft tissue layer 112 within ROI 115 through precise spatial and temporal control of acoustic energy deposition, i.e., control of ultrasound probe 105 is confined within selected time and space parameters, with such control being independent of the tissue. In accordance with one embodiment, control system and ultrasound probe 105 can be configured for spatial control of therapeutic ultrasound energy 120 by controlling the manner of distribution of the therapeutic ultrasound energy 120. For example, spatial control may be realized through selection of the type of one or more transducer configurations insonifying ROI 115, selection of the placement and location of ultrasound probe 105 for delivery of therapeutic ultrasound energy 120 relative to ROI 115 e.g., ultrasound probe 105 being configured for scanning over part or whole of ROI 115 to produce contiguous thermal injury having a particular orientation or otherwise change in distance from ROI 115, and/or control of other environment parameters, e.g., the temperature at the acoustic coupling interface can be controlled, and/or the coupling of ultrasound probe 105 to human tissue. In addition to the spatial control parameters, control system and ultrasound probe 105 can also be configured for temporal control, such as through adjustment and optimization of drive amplitude levels, frequency/waveform selections, e.g., the types of pulses, bursts or continuous waveforms, and timing sequences and other energy drive characteristics to control thermal ablation of tissue. The spatial and/or temporal control can also be facilitated through open-loop and closed-loop feedback arrangements, such as through the monitoring of various spatial and temporal characteristics. As a result, control of acoustical energy within six degrees of freedom, e.g., spatially within the X, Y and Z domain, as well as the axis of rotation within the XY, YZ and XZ domains, can be suitably achieved to generate conformal lesions 25 of variable shape, size and orientation.

For example, through such spatial and/or temporal control, ultrasound probe 105 can enable the regions of thermal injury to possess arbitrary shape and size and allow the tissue to be destroyed (ablated) in a controlled manner. With reference to Figure 8, one or more thermal lesions may be created within fibrous soft tissue layer 112, with such thermal lesions 25 having a narrow or wide lateral extent, long or short axial length, and/or deep or shallow placement in muscle and connective tissue 112. For example, cigar shaped lesions may be produced in a vertical disposition 204 and/or horizontal disposition 206. In addition, raindrop-shaped lesions 208, flat planar lesions 210, round lesions 212 and/or other v-shaped/ellipsoidal lesions 214 may be formed, among others. For example, mushroom-shaped lesion 220 may be provided, such as through initial generation of an initial round or cigar-shaped lesion 222, with continued application of therapeutic ultrasound energy 120 resulting in thermal expansion to further generate a growing lesion 224, such thermal expansion being continued until mushroom-shaped lesion 220 is achieved. The plurality of shapes can also be configured in various sizes and orientations, e.g., lesions 208 could be rotationally oriented clockwise or counterclockwise at any desired angle, or made larger or smaller as selected, all depending on spatial and/or temporal control. Moreover, separate islands of destruction, i.e., multiple lesions separated throughout fibrous soft tissue layer 112, may also be created over part of or the whole portion within ROI 115. In addition, contiguous structures and/or overlapping structures 216 may be provided from the controlled configuration of discrete lesions. For example, a series of one or more crossed-lesions 218 can be generated along a tissue region to facilitate various types of treatment methods.

The specific configurations of controlled thermal injury are selected to achieve the desired tissue and therapeutic effect. For example, any tissue effect can be realized, including but not limited to thermal and non-thermal streaming, cavitational, hydrodynamic, ablative, hemostatic, diathermic, and/or resonance-induced tissue effects. Additional embodiments useful for creating lesions may be found in US Patent Publication No. 20060116671 entitled "Method and System for Controlled Thermal Injury of Human Superficial Tissue" published June 1, 2006,

Now with reference to Figure 9, treatment system 148, according to various embodiments, is illustrated. In various embodiments, treatment system comprises controller 144, display 146, ultrasound probe 105, and interface 142 for communication between ultrasound probe 105 and controller 114. Ultrasound probe 105 may be controlled and operated by controller 144, which also relays and processes images obtained by ultrasound probe 105 to display 146. In one embodiment, controller 144 is capable of coordination and control of the entire treatment process to achieve the desired therapeutic effect on fibrous soft tissue layer 112 within ROI 115. For example, in one embodiment, controller 144 may comprise power source components, sensing and monitoring components, cooling and coupling controls, and/or processing and control logic components. Controller 144 may be configured and optimized in a variety of ways with more or less subsystems and components to implement treatment system 148 for controlled targeting of a portion of fibrous soft tissue layer 112, and the exemplary embodiment in Figure 9 is merely for illustration purposes.

For example, for power sourcing components, controller 144 may comprise one or more direct current (DC) power supplies capable of providing electrical energy for the entire controller 144, including power required by a transducer electronic amplifier/driver. A DC current sense device may also be provided to confirm the level of power entering amplifiers/drivers for safety and monitoring purposes.

In one embodiment, amplifiers/drivers may comprise multi-channel or single channel power amplifiers and/or drivers. In one embodiment for transducer array configurations, amplifiers/drivers may also be configured with a beamformer to facilitate array focusing. One beamformer may be electrically excited by an oscillator/digitally controlled waveform synthesizer with related switching logic.

Power sourcing components may also comprise various filtering configurations. For example, switchable harmonic filters and/or matching may be used at the output of amplifier/driver to increase the drive efficiency and effectiveness. Power detection components may also be included to confirm appropriate operation and calibration. For example, electric power and other energy detection components may be used to monitor the amount of power entering ultrasound probe 105.

Various sensing and monitoring components may also be implemented within controller 144. For example, in one embodiment, monitoring, sensing, and interface control components may be capable of operating with various motion detection systems implemented within ultrasound probe 105, to receive and process information such as acoustic or other spatial and temporal information from ROI 115. Sensing and monitoring components may also comprise various controls, interfacing, and switches and/or power detectors. Such sensing and monitoring components may facilitate open-loop and/or closed-loop feedback systems within treatment system 146.

In one embodiment, sensing and monitoring components 62 may further comprise a sensor that may be connected to an audio or visual alarm system to prevent overuse of system. In this exemplary embodiment, the sensor may be capable of sensing the amount of energy transferred to the skin, and/or the time that treatment system 146 has been actively emitting energy. When a certain time or temperature threshold has been reached, the alarm may sound an audible alarm, or cause a visual indicator to activate to alert the user that a threshold has been reached. This may prevent overuse of treatment system 146. In one embodiment, the sensor may be operatively connected to controller 144 and force controller 144, to stop emitting therapeutic ultrasound energy 120 from ultrasound probe 105.

Additionally, one controller 144 may further comprise a system processor and various digital control logic, such as one or more of microcontrollers, microprocessors, fieldprogrammable gate arrays, computer boards, and associated components, including firmware and control software, which may be capable of interfacing with user controls and interfacing circuits as well as input/output circuits and systems for communications, displays, interfacing, storage, documentation, and other useful functions. System software may be capable of controlling all initialization, timing, level setting, monitoring, safety monitoring, and all other system functions required to accomplish user-defined treatment objectives. Further, various control switches may also be suitably configured to control operation.

With reference again to Figure 9, one treatment system 148 also may comprise display 146 capable of providing images of ROI 115 in various embodiments where ultrasound energy may be emitted from ultrasound probe 105 in a manner for imaging. In one embodiment, display 146 is a computer monitor. Display 146 may be capable of enabling the user to facilitate localization of treatment area and surrounding structures, for example, identification of fibrous soft tissue layer 112. In an alternative exemplary embodiment, the user may know the location of the specific fibrous soft tissue layer 112 to be treated based at least in part upon prior experience or education and without display 146.

After localization, therapeutic ultrasound energy 120 is delivered at a depth, distribution, timing, and energy level to achieve the desired therapeutic effect at ROI 115 to treat injury. Before, during and/or after delivery of therapeutic ultrasound energy 120, monitoring of the treatment area and surrounding structures may be conducted to further plan and assess the results and/or provide feedback to controller 148, and to a system operator via display 146. In one embodiment, localization may be facilitated through ultrasound imaging that may be used to define the position of injury location and/or fibrous soft tissue layer 112 in ROI 115.

Feedback information may be generated or provided by any one or more acoustical sources, such as B-scan images, A-lines, Doppler or color flow images, surface acoustic wave devices, hydrophones, elasticity measurement, or shear wave based devices. In addition, optical sources can also be utilized, such as video and/or infrared cameras, laser Doppler imagers, optical coherence tomography imagers, and temperature sensors. Further, feedback information can also be provided by semiconductors, such as thermistors or solid state temperature sensors, by electronic and electromagnetic sensors, such as impedance and capacitance measurement devices and/or thermocouples, and by mechanical sensors, such as stiffness gages, strain gages or stress measurement sensors, or any suitably combination thereof. Moreover, various other switches, acoustic or other sensing mechanisms and methods may be employed to enable transducer 75 to be acoustically coupled to one or more ROI 115.

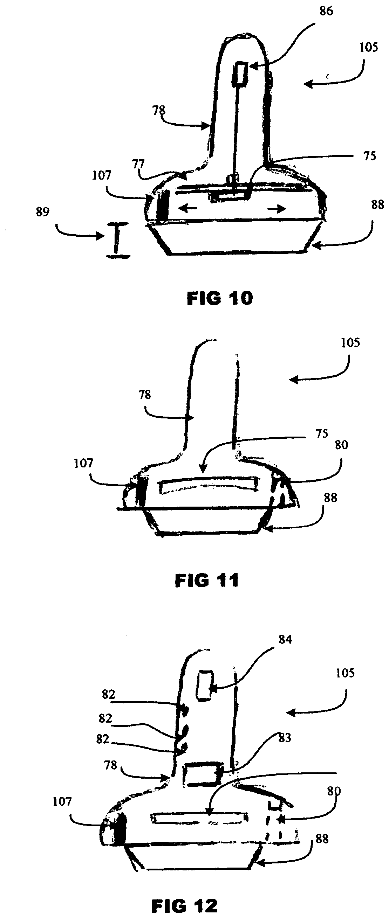

Moving to Figure 10, ultrasound probe comprising a transducer and a motion mechanism, according to various embodiments, is illustrated. In various embodiments, ultrasound probe 105 comprises transducer 75. In some embodiments, ultrasound probe 105 comprises motion mechanism 77, which moves transducer 75 along a plane substantially parallel to sink surface 104. Motion mechanism 77 can be coupled to motor 86. Motion mechanism 77 can be controlled by controller 144. In some embodiments, ultrasound probe 105 comprises position sensor 107, as described herein. In some embodiments, transducer 75 is a single element operable for imaging and emitting therapeutic ultrasound energy 120, as described herein. In some embodiments, transducer 75 is a multi-element array operable for imaging and emitting therapeutic ultrasound energy 120, as described herein. However, in some embodiments, transducer 75 is operable for emitting therapeutic ultrasound energy 120 and is not operable for imaging, as described herein.

In various embodiments, transducer 75, motion mechanism 77, motor 87, optionally position sensor 107 can held within enclosure 78. In one embodiment, enclosure 78 is designed for comfort and control while used in an operator's hand. Enclosure 78 may also contain various electronics, EEPROM, interface connection, and/or ram for holding programs. In various embodiments, ultrasound probe 105 comprises tip 88. In some embodiments, tip 88 is gel and/or liquid filled. Tip can include EEPROM which is in communication with at least one of electronics in ultrasound probe 105 and controller 144. Data for EEPROM can be collected in controller 144 and connected to treatment data. In one embodiment, tip is disposable, and for example EEPROM determines if tip has been used and will not allow treatment to begin tip 88 that has been previously used. In some embodiments, tip 88 has height 89 which can control therapeutic ultrasound energy 120 depth into fibrous soft tissue layer 112. In some embodiments, a plurality of tips 88, each having a different height 89 may be used to direct therapeutic ultrasound energy 120 to a plurality of depths in fibrous soft tissue layer 112.

Transducer 75 may further comprise a reflective surface, tip 88, or area at the end of the transducer 75 that emits therapeutic ultrasound energy 120. This reflective surface may enhance, magnify, or otherwise change therapeutic ultrasound energy 120 emitted from ultrasound probe 105.

Therapeutic ultrasound energy 120 from transducer 75 may be spatially and/or temporally controlled at least in part by changing the spatial parameters of transducer 75, such as the placement, distance, treatment depth and transducer 75 structure, as well as by changing the temporal parameters of transducer 75, such as the frequency, drive amplitude, and timing, with such control handled via controller 144. Such spatial and temporal parameters may also be monitored in open-loop and/or closed-loop feedback systems within treatment system 148.

In various embodiments, ultrasound probe 105 comprises a transducer 75 capable of emitting therapeutic ultrasound energy 120 into ROI 115. This may heat ROI 115 at a specific depth to target fibrous soft tissue layer 112 causing that tissue to be ablated, microablated, coagulated, incapacitated, partially incapacitated, rejuvenated, shortened, paralyzed, or removed.

A coupling gel may be used to couple ultrasound probe 105 to ROI 115 at skin surface 104. Therapeutic ultrasound energy 120 may be emitted in various energy fields in this exemplary embodiment. In this exemplary embodiment, the energy fields may be focused, defocused, and/or made substantially planar by transducer 75, to provide many different effects. Energy may be applied in a C-plane or C-scan. For example, in one exemplary embodiment, a generally substantially planar energy field may provide a heating and/or pretreatment effect, a focused energy field may provide a more concentrated source of heat or hypothermal effect, and a non-focused energy field may provide diffused heating effects. It should be noted that the term "non-focused" as used throughout encompasses energy that is unfocused or defocused.

In various embodiments, transducer 75 may comprise one or more transducers elements for facilitating treatment. Transducer 75 may further comprise one or more transduction elements. Transduction element may comprise piezoelectrically active material, such as lead zirconante titanate (PZT), or other piezoelectrically active material such as, but not limited to, a piezoelectric ceramic, crystal, plastic, and/or composite materials, as well as lithium niobate, lead titanate, barium titanate, and/or lead metaniobate. In addition to, or instead of, a piezoelectrically active material. Transducer 75 may comprise any other materials configured for generating radiation and/or acoustical energy. Transducer 75 may also comprise one or more matching and/or backing layers configured along with the transduction element, such as being coupled to the piezoelectrically active material. Transducer 75 may also be configured with single or multiple damping elements along the transduction element.

In one embodiment, the thickness of the transduction element of transducer 75 may be configured to be uniform. That is, the transduction element may be configured to have a thickness that is generally substantially the same throughout. In another exemplary embodiment, the transduction element may also be configured with a variable thickness, and/or as a multiple damped device. For example, the transduction element of transducer 75 may be configured to have a first thickness selected to provide a center operating frequency of a lower range, for example from approximately 1 kHz to 3 MHz. The transduction element may also be configured with a second thickness selected to provide a center operating frequency of a higher range, for example from approximately 3 to 100 MHz or other frequency ranges described herein.

In yet another exemplary embodiment, transducer 75 may be configured as a single broadband transducer excited with two or more frequencies to provide an adequate output for raising the temperature within ROI 115 to the desired level. Transducer 75 may also be configured as two or more individual transducers, wherein each transducer 75 may comprise a transduction element. The thickness of the transduction elements may be configured to provide center-operating frequencies in a desired treatment range. For example, in one embodiment, transducer 75 may comprise a first transducer 75 configured with a first transduction element having a thickness corresponding to a center frequency range of approximately 1 MHz to 3 MHz, and a second transducer 75 configured with a second transduction element having a thickness corresponding to a center frequency of approximately 3 MHz to 100 MHz or frequency ranges described herein.

Moreover, in some embodiments, any variety of mechanical lenses or variable focus lenses, e.g. liquid-filled lenses, may also be used to focus and or defocus the energy field. For example, transducer 75 may also be configured with an electronic focusing array in combination with one or more transduction elements to facilitate increased flexibility in treating ROI 115. Array may be configured in a manner similar to transducer 75. That is, array may be configured as an array of electronic apertures that may be operated by a variety of phases via variable electronic time delays. Accordingly, the electronic apertures of array may be manipulated, driven, used, configured to produce and/or deliver energy in a manner corresponding to the phase variation caused by the electronic time delay. For example, these phase variations may be used to deliver defocused beams, planar beams, and/or focused beams, each of which may be used in combination to achieve different physiological effects in ROI 115.

Transduction elements may be configured to be concave, convex, and/or planar. For example, transduction elements can be configured to be concave in order to provide focused energy for treatment of ROI 115. In another exemplary embodiment, transduction elements may be configured to be substantially flat in order to provide substantially uniform energy to ROI 115. In addition, transduction elements may be configured to be any combination of concave, convex, and/or substantially flat structures. For example, a first transduction element may be configured to be concave, while a second transduction element may be configured to be substantially flat.

Moreover, transduction element can be any distance from the skin surface 104. In that regard, it can be far away from the skin surface 104 disposed within a long transducer 75 or it can be just a few millimeters from skin surface 104. In certain exemplary embodiments, positioning the transduction element closer to skin surface 104 is better for emitting ultrasound at high frequencies. Moreover, both two and three dimensional arrays of transduction elements can be used in various embodiments.

In some embodiments, transducer 75 may also be configured as an annular array to provide planar, focused and/or defocused acoustical energy. For example, in one embodiment, an annular array may comprise a plurality of rings. Rings may be mechanically and electrically isolated into a set of individual elements, and may create planar, focused, or defocused waves. For example, such waves can be centered on-axis, such as by methods of adjusting corresponding phase delays. An electronic focus may be moved along various depth positions in ROI 115, and may enable variable strength or beam tightness, while an electronic defocus may have varying amounts of defocusing. In one embodiment, a lens and/or convex or concave shaped annular array may also be provided to aid focusing or defocusing such that any time differential delays can be reduced. Movement of annular array in one, two or three-dimensions, or along any path, such as through use of probes, motion mechanisms, any conventional robotic arm mechanisms, and the like may be implemented to scan and/or treat a volume or any corresponding space within ROI 115.

In one embodiment, suction is used to attach ultrasound probe 105 to the patient's body. In this exemplary embodiment, a negative pressure differential is created and ultrasound probe 105 attaches to skin surface 104 by suction. A vacuum-type device is used to create the suction and the vacuum device can be integral with, detachable, or completely separate from ultrasound probe 105. The suction attachment of ultrasound probe 105 to skin surface 104 and associated negative pressure differential ensures that ultrasound probe 105 is properly coupled to skin surface 104. Further, the suction-attachment also reduces the thickness of the tissue to make it easier to reach ROI 115. In some embodiments, a coupling gel is used to couple ultrasound probe 105 to ROI 115, as described herein. The coupling gel can include medicines and other drugs, such as, for example, medicants, as described herein, and the application of therapeutic ultrasound energy 120 can facilitate transdermal drug delivery to ROI 115 and surrounding tissue.

In some embodiments, a cooling/coupling control system may be provided, and may be capable of removing waste heat from ultrasound probe 105. Furthermore the cooling/coupling control system may be capable of providing a controlled temperature at skin surface 104 and deeper into tissue, and/or provide acoustic coupling from ultrasound probe 105 to ROI 115. Such cooling/coupling control systems can also be capable of operating in both open-loop and/or closed-loop feedback arrangements with various coupling and feedback components.

With reference to Figure 11, an ultrasound probe comprising a transducer, according to various embodiments, is illustrated. In various embodiments, ultrasound probe 105 comprises transducer 75. In some embodiments, ultrasound probe 105 comprises imaging transducer 80. In some embodiments, ultrasound probe 105 comprises position sensor 107, as described herein. In some embodiments, transducer 75 is operable for emitting therapeutic ultrasound energy 120 and imaging transducer 80 is operable for imaging, as described herein.

In various embodiments, transducer 75, imaging transducer 80, and optionally position sensor 107, can be held within enclosure 78. In one embodiment, enclosure 78 is designed for comfort and control while used in an operator's hand. Enclosure 78 may also contain various electronics, EEPROM, interface connection, and/or ram for holding programs. In various embodiments, ultrasound probe 105 comprises tip 88. In some embodiments, tip 88 is gel and/or liquid filled. Tip can include EEPROM which is in communication with at least one of electronics in ultrasound probe 105 and controller 144. Data for EEPROM can be collected in controller 144 and connected to treatment data. In one embodiment, tip is disposable, and for example EEPROM determines if tip has been used and will not allow treatment to begin tip 88 that has been previously used. In some embodiments, tip 88 has height 89 which can control therapeutic ultrasound energy 120 depth into fibrous soft tissue layer 112. In some embodiments, a plurality of tips 88, each having a different height 89 may be used to direct therapeutic ultrasound energy 120 to a plurality of depths in fibrous soft tissue layer 112.

Transducer 75 may further comprise a reflective surface, tip 88, or area at the end of the transducer 75 that emits therapeutic ultrasound energy 120. This reflective surface may enhance, magnify, or otherwise change therapeutic ultrasound energy 120 emitted from ultrasound probe 105.

With reference to Figure 12, a hand held ultrasound probe, according to various embodiments, is illustrated. In various embodiments, ultrasound transducer 105 comprises transducer 75, as described herein, and may be controlled and operated by a hand-held format control system. An external battery charger can be used with rechargeable-type batteries 84 or the batteries 84 can be single-use disposable types, such as M-sized cells. Power converters produce voltages for powering a driver/feedback circuit with tuning network driving transducer 75. Ultrasound probe 105 is coupled to skin surface 104 via one or more tips 88, which can be composed of at least one of a solid media, semi-solid e.g. gelatinous media, and/or liquid media equivalent to an acoustic coupling agent (contained within a housing). Tip 88 is coupled to skin surface 104 with an acoustic coupling agent. In addition, a microcontroller and timing circuits with associated software and algorithms provide control and user interfacing via a display or LED-type indicators 83, and other input/output controls 82, such as switches and audio devices. A storage element, such as an Electrically Erasable Programmable Read-Only Memory ("EEPROM"), secure EEPROM, tamper-proof EEPROM, or similar device can hold calibration and usage data. A motion mechanism with feedback can be controlled to scan the transducer 75in a linear pattern or a two-dimensional pattern or over a varied depth. Other feedback controls comprise capacitive, acoustic, or other coupling detection means, limiting controls, and thermal sensor. EEPROM can be coupled with at least one of tip 88, transducer 75, thermal sensor, coupling detector, and tuning network. Data for EEPROM can be collected in controller 144 and connected to treatment data.

Ultrasound transducer can comprise tip 88 that can be disposed of after contacting a patient and then replaced for sanitary reasons. In one embodiment, tip is disposable, and for example EEPROM determines if tip has been used and will not allow treatment to begin tip 88 that has been previously used. In some embodiments, tip 88 has height 89 which can control therapeutic ultrasound energy 120 depth into fibrous soft tissue layer 112. In some embodiments, a plurality of tips 88, each having a different height 89 may be used to direct therapeutic ultrasound energy 120 to a plurality of depths in fibrous soft tissue layer 112.

Transducer 75 may further comprise a reflective surface, tip 88, or area at the end of the transducer 75 that emits therapeutic ultrasound energy 120. This reflective surface may enhance, magnify, or otherwise change therapeutic ultrasound energy 120 emitted from ultrasound probe 105.

In some embodiments, ultrasound probe 105 comprises imaging transducer 80. In some embodiments, ultrasound probe 105 comprises position sensor 107, as described herein. In some embodiments, transducer 75 is operable for emitting therapeutic ultrasound energy 120 and imaging transducer 80 is operable for imaging, as described herein.

In various embodiments, ultrasound probe 105 comprises transducer 75. In some embodiments, ultrasound probe 105 comprises position sensor 107, as described herein. In some embodiments, transducer 75 is a single element operable for imaging and emitting therapeutic ultrasound energy 120, as described herein. In some embodiments, transducer 75 is a multi-element array operable for imaging and emitting therapeutic ultrasound energy 120, as described herein. However, in some embodiments, transducer 75 is operable for emitting therapeutic ultrasound energy 120 and is not operable for imaging, as described herein.

In various embodiments, transducer 75, and optionally position sensor 107 can held within enclosure 78. In one embodiment, enclosure 78 is designed for comfort and control while used in an operator's hand. Enclosure 78 may also contain various electronics, EEPROM, interface connection, motion mechanisms, and/or ram for holding programs.

In various embodiments, ultrasound probe 105 can be in communication with wireless device via wireless interface. Typically, wireless device has display and a user interface such as, for example, a keyboard. Examples of wireless device can include but are not limited to: personal data assistants ("PDA"), cell phone, iphone, ipad, computer, laptop, netbook, or any other such device now known or developed in the future. Examples of wireless interface include but are not limited to any wireless interface described herein and any such wireless interface now known or developed in the future. Accordingly, ultrasound probe 105 comprises any hardware, such as, for example, electronics, antenna, and the like, as well as, any software that may be used to communicate via wireless interface.

In various embodiments, wireless device can display an image generated by handheld probe 105. In various embodiments, wireless device can control handheld ultrasound probe 105. In various embodiments, wireless device can store data generated by handheld ultrasound probe 105.

Therapeutic ultrasound energy 120 from transducer 75 may be spatially and/or temporally controlled at least in part by changing the spatial parameters of transducer 75, such as the placement, distance, treatment depth and transducer 75 structure, as well as by changing the temporal parameters of transducer 75, such as the frequency, drive amplitude, and timing, with such control handled via controller in hand-held assembly of ultrasound probe 105. In various embodiments, ultrasound probe 105 comprises a transducer 75 capable of emitting therapeutic ultrasound energy 120 into ROI 115. This may heat ROI 115 at a specific depth to target fibrous soft tissue layer 112 causing that tissue to be ablated, microablated, coagulated, incapacitated, partially incapacitated, rejuvenated, shortened, paralyzed, or removed.

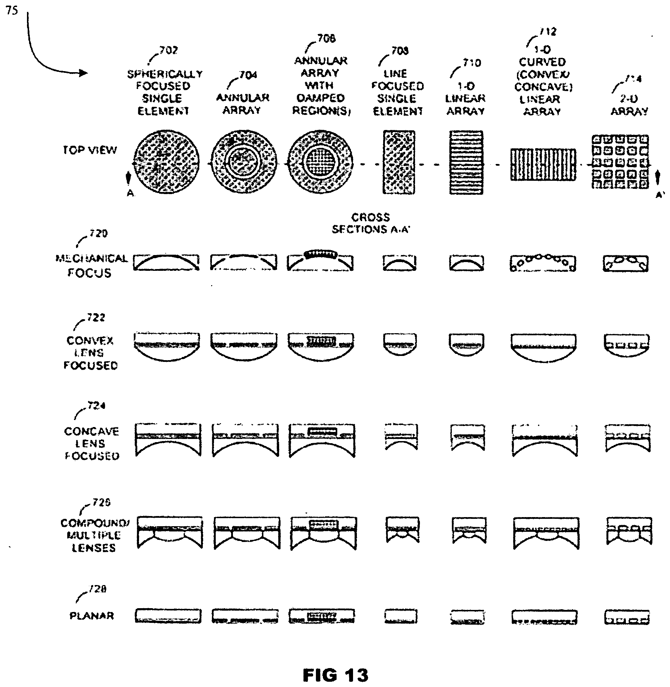

Referring to Figure 13, a plurality of exemplary transducer configurations, according to various embodiments, is illustrated. In some embodiments, transducer 75 can be configured to comprise a spherically focused single element 36, annular/multi-element 38, annular with imaging region(s) 40, line-focused single element 42, 1-D linear array 44, 1-D curved (convex/concave) linear array 46, or 2-D array 48. With further reference to Figure 7, any of the previous described configuration of transducer 75 can be coupled to one of mechanical focus 50, convex lens focus 52, concave lens focus 54, compound/multiple lens focused 56, or planar array form 58, and combinations thereof. Such transducer 75 configurations individually or coupled to a focusing element can achieve focused, unfocused, or defocused sound fields for at least one of imaging and therapy.

As described herein, various embodiments provide methods of treating sport related injuries. In some embodiments, the methods include targeting a ROI 115 comprising fibrous soft tissue layer 112, directing therapeutic ultrasound energy 120 to the ROI 115, producing a therapeutic effect to a portion of the fibrous soft tissue layer 112, thereby improving or healing the injury. Further examples of methods directed to specific injuries are discussed herein, in accordance to various embodiments.

In various embodiments, methods, described herein, can stimulate coagulation by depositing target ultrasound energy with or without a medicant. Coagulation is a complex process by which blood forms clots. It is an important part of hemostasis (the cessation of blood loss from a damaged vessel), wherein a damaged blood vessel wall is covered by a platelet and fibrin-containing clot to stop bleeding and begin repair of the damaged vessel. Disorders of coagulation can lead to an increased risk of bleeding (hemorrhage) or obstructive clotting (thrombosis).

Coagulation begins almost instantly after an injury to the blood vessel has damaged the endothelium (lining of the vessel). Exposure of the blood to proteins such as tissue factor initiates changes to blood platelets and the plasma protein fibrinogen, a clotting factor. Platelets immediately form a plug at the site of injury; this is called primary hemostasis. Secondary hemostasis occurs simultaneously: Proteins in the blood plasma, called coagulation factors or clotting factors, respond in a complex cascade to form fibrin strands, which strengthen the platelet plug.

In some embodiments, methods, described herein, can initiate coagulation cascade by depositing target ultrasound energy with or without a medicant. The coagulation cascade of secondary hemostasis has two pathways which lead to fibrin formation. These are the contact activation pathway (formerly known as the intrinsic pathway), and the tissue factor pathway (formerly known as the extrinsic pathway). It was previously thought that the coagulation cascade consisted of two pathways of equal importance joined to a common pathway. It is now known that the primary pathway for the initiation of blood coagulation is the tissue factor pathway. The pathways are a series of reactions, in which a zymogen (inactive enzyme precursor) of a serine protease and its glycoprotein co-factor are activated to become active components that then catalyze the next reaction in the cascade, ultimately resulting in cross-linked fibrin.

The coagulation factors are generally serine proteases (enzymes). There are some exceptions. For example, FVIII and FV are glycoproteins, and Factor XIII is a transglutaminase. Serine proteases act by cleaving other proteins at specific sites. The coagulation factors circulate as inactive zymogens. The coagulation cascade is classically divided into three pathways. The tissue factor and contact activation pathways both activate the "final common pathway" of factor X, thrombin and fibrin.

Soon after injury, a wound healing cascade is unleashed. This cascade is usually said to take place in three phases: the inflammatory, proliferative, and maturation stages.

In some embodiments, methods, described herein, can peak inflammation by depositing target ultrasound energy with or without a medicant. In the inflammatory phase, macrophages and other phagocytic cells kill bacteria, debride damaged tissue and release chemical factors such as growth hormones that encourage fibroblasts, epithelial cells and endothelial cells which make new capillaries to migrate to the area and divide.

In the proliferative phase, immature granulation tissue containing plump active fibroblasts forms. Fibroblasts quickly produce abundant type III collagen, which fills the defect left by an open wound. Granulation tissue moves, as a wave, from the border of the injury towards the center.

As granulation tissue matures, the fibroblasts produce less collagen and become more spindly in appearance. They begin to produce the much stronger type I collagen. Some of the fibroblasts mature into myofibroblasts which contain the same type of actin found in smooth muscle, which enables them to contract and reduce the size of the wound.

During the maturation phase of wound healing, unnecessary vessels formed in granulation tissue are removed by apoptosis, and type III collagen is largely replaced by type I. Collagen which was originally disorganized is cross-linked and aligned along tension lines. This phase can last a year or longer. Ultimately a scar made of collagen, containing a small number of fibroblasts is left.

In various embodiments, methods described herein can treat either recent or older injuries, or combinations thereof. Inflammation can be classified as either acute or chronic. Acute inflammation is the initial response of the body to harmful stimuli and is achieved by the increased movement of plasma and leukocytes (especially granulocytes) from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells present at the site of inflammation and is characterized by simultaneous destruction and healing of the tissue from the inflammatory process. In various embodiments, methods can treat chronic inflammation. In various embodiments, methods can treat acute inflammation. In some embodiments, method 100 can treat a combination of acute and chronic inflammation. In one embodiment, methods described herein can treat scar material in tissue at an older injury site. In one embodiment, methods described herein can treat an abscess in tissue at an older injury site. In one embodiment, methods described herein can treat damaged tissue at an older injury site.

In one outcome of inflammation and healing, fibrosis can occur. Large amounts of tissue destruction, or damage in tissues unable to regenerate, cannot be regenerated completely by the body. Fibrous scarring occurs in these areas of damage, forming a scar composed primarily of collagen. The scar will not contain any specialized structures, such as parenchymal cells, hence functional impairment may occur.

According to various embodiments, methods can include non-invasive shrinkage or removal of a fibrous scar located in fibrous soft tissue layer 112. Such a method can include targeting the fibrous scar in ROI 115, directing ablative ultrasound energy to the fibrous scar, ablating at least a portion of the fibrous scar, and shrinking or removing the fibrous scar. The method can also include imaging the fibrous scar. The method can also include imaging the scar after the ablating at least a portion of the fibrous scar. The method can include comparing a measurement of the scar before and after the ablating step. The method can include directing acoustical pressure or cavitation to the scar after the ablating step to further break up the scar. The method can include increasing blood perfusion to the ROI 115. The method can also include any of the steps of method 100.

In another outcome of inflammation and healing, an abscess can be formed. A cavity is formed containing pus, which is a liquid comprising dead white blood cells, and bacteria mixed with destroyed cells. According to various embodiments, methods can include noninvasive removal of an abscess located in fibrous soft tissue layer 112. Such a method can include targeting the abscess in ROI 115, directing ablative ultrasound energy to the abscess, ablating at least a portion of the abscess, and shrinking or removing the abscess. The method can also include imaging the abscess. The method can also include imaging the abscess after the ablating at least a portion of the abscess. The method can include comparing a measurement of the abscess before and after the ablating step. The method can include directing acoustical pressure or cavitation to the scar after the ablating step to further break up the abscess. The method can include destroying bacteria located in the abscess. The method can include increasing blood perfusion to the ROI 115. The method can include administering a medicant to the ROI 115. The method can also include any of the steps of method 100.

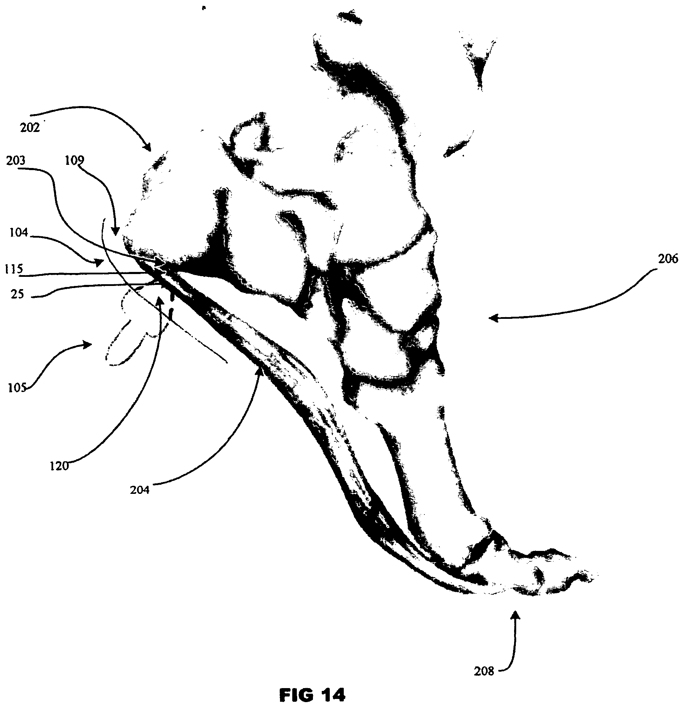

Now with reference to Figure 14, plantar fasciitis is a painful condition caused by overuse of the plantar fascia 204 or arch tendon of the foot. The plantar fascia 204 is a broad, thick band of tissue that runs from under the heel 202 to the front of the foot 206. Plantar fasciitis is traditionally thought to be an inflammatory condition. The cause of pain and dysfunction is now thought to be degeneration of the collagen fibers 203 close to the attachment to the calcaneus (heel bone).

According to various embodiments, methods of treating plantar fascia are provided. Such a method can include targeting the collagen fibers 203 within the fascia 204 in ROI 115, directing therapeutic ultrasound energy 210 to the inflammatory cells within the fascia, ablating at least a portion of the collagen fibers 203 within the fascia 204, and improving collagen fibers 203. The method can include coupling ultrasound probe 105 to ROI 115. The method can include focusing therapeutic ultrasound energy 120 to create a lesion 25 in a portion of collagen fibers 203. The method can include creating a plurality of lesions 25 in collagen fibers 203. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to collagen fibers 203. The method can also include imaging collagen fibers 203 within the fascia 204. The method can also include imaging collagen fibers 203 after the ablating at least a portion of collagen fibers 203. The method can include comparing a measurement of collagen fibers 203 within the fascia 204 before and after the ablating step. The method can include directing acoustical pressure or cavitation to the collagen fibers 203 after the ablating step further improving collagen fibers 203. The method can include increasing blood perfusion to the ROI 115. The method can include administering a medicant to the ROI 115. The method can also include any of the steps of method 100.

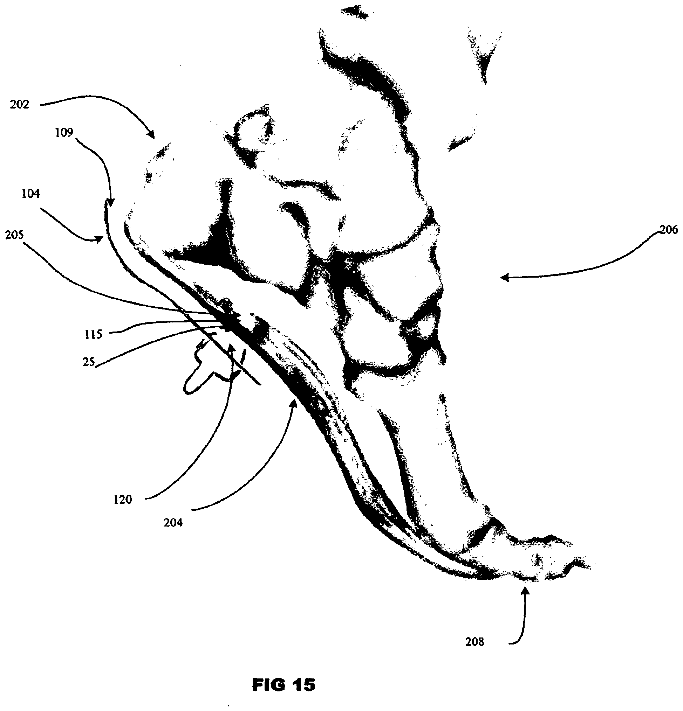

Now referencing Figure 15, the plantar fascia 204 or arch ligament is a band that runs from under the heel to the front of the foot 206. A strain or partial rupture 205 to this is quite common. A plantar fascia strain might result from one single traumatic incident (an acute injury) or may gradually occur over a period of time (a chronic injury).

According to various embodiments, methods of treating plantar fascia strains or partial ruptures 205 are provided. Such a method can include targeting the strain or partial rupture 205 within the fascia 204 in ROI 115, directing therapeutic ultrasound energy 120 to the strain or partial rupture 205, ablating at least a portion of the strain or partial rupture 205, and improving strain or partial rupture 205 within the fascia 204. The method can include coupling ultrasound probe 105 to ROI 115. The method can include focusing therapeutic ultrasound energy 120 to create a lesion 25 in a portion of the strain or partial rupture 205. The method can include creating a plurality of lesions 25 in the strain or partial rupture 205. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the strain or partial rupture 205. The method can also include imaging the strain or partial rupture 205 within the fascia 204. The method can also include imaging the strain or partial rupture 205 within the fascia 204 after the ablating at least a portion of the strain or partial rupture 205. The method can include comparing a measurement of the strain or partial rupture 205 within the fascia 204 before and after the ablating step. The method can include directing acoustical pressure or cavitation to the strain or partial rupture 205 after the ablating step further improving strain or partial rupture 205 within the fascia 204. The method can include welding the partial rupture 205 with therapeutic ultrasound energy 120. The method can include increasing blood perfusion to the ROI 115. The method can include administering a medicant to the ROI 115. The method can also include any of the steps of method 100.

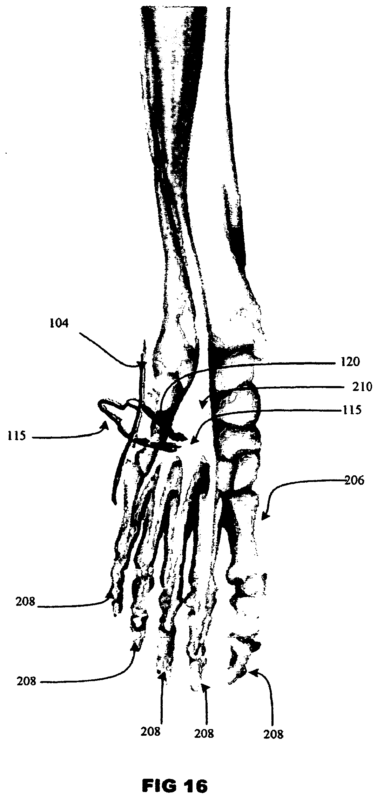

In Figure 16, the tendons which straighten the toes 208 run along the top of the foot 206 and are known as the extensor tendons 210. They are the tibialis anterior, extensor hallucis longus, extensor hallucis brevis, extensor digitorum longus and extensor digitorum brevis. These tendons can become inflamed causing pain and swelling. Inflammation of the tibialis anterior is most common but inflammation of the extensor digitorum muscles is rare.

According to various embodiments, methods of treating inflamed extensor tendons are provided. Such a method can include targeting the inflamed extensor tendon in ROI 115, directing therapeutic ultrasound energy 120 to the inflamed extensor tendon, ablating at least a portion of the inflamed extensor tendon, and improving inflamed extensor tendon. The method can include coupling ultrasound probe 105 to ROI 115. The method can include focusing therapeutic ultrasound energy 120 to create a lesion 25 in a portion of the inflamed extensor tendon. The method can include creating a plurality of lesions 25 in the inflamed extensor tendon. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed extensor tendon. The method can also include imaging the inflamed extensor tendon. The method can also include imaging the inflamed extensor tendon after the ablating at least a portion of the inflamed extensor tendon. The method can include comparing a measurement of the inflamed extensor tendon the fascia before and after the ablating step. The method can include directing acoustical pressure or cavitation to the inflamed extensor tendon after the ablating step further improving inflamed extensor tendon. The method can include increasing blood perfusion to the ROI 115. The method can include administering a medicant to the ROI 115. The method can also include any of the steps of method 100.

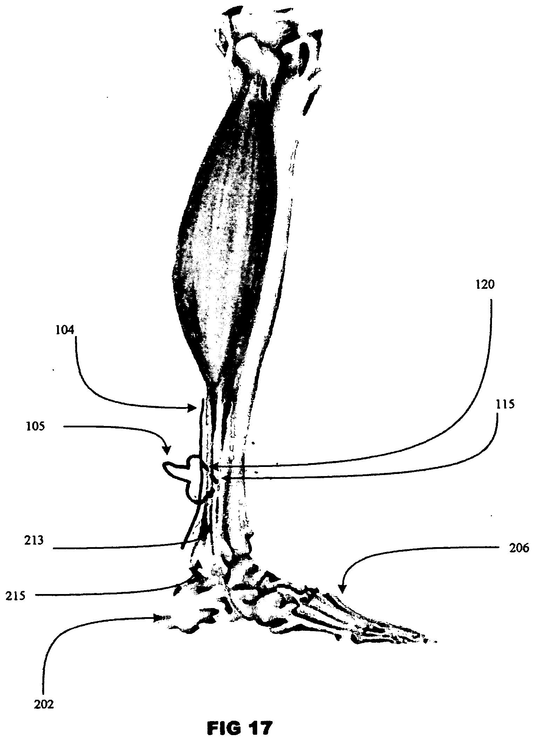

Referring to Figure 17, the peroneal tendon 213 runs behind the lateral malleolus or the bony bit on the outside of the ankle 215. The peroneus longus tendon runs around the back of the lateral malleolus and under the foot 206 to attach at the outside of the first metatarsal and cuniform bones. The peroneus brevis passes around the back of the lateral malleolus and attaches to the outside of the foot 206 on the base of the 5th metatarsal. The muscles act to plantar flex the foot 206 and evert the foot 206. Peroneal tendinopathy can cause pain and swelling in the ankle 213 or heel 202.

According to various embodiments, methods of treating an inflamed peroneal tendon are provided. Such a method can include targeting the inflamed peroneal tendon in ROI 115, directing therapeutic ultrasound energy 120 to the inflamed peroneal tendon, ablating at least a portion of the inflamed peroneal tendon, and improving inflamed peroneal tendon. The method can include coupling ultrasound probe 105 to ROI 115. The method can include focusing therapeutic ultrasound energy 120 to create a lesion 25 in a portion of the inflamed peroneal tendon. The method can include creating a plurality of lesions 25 in the inflamed peroneal tendon. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed peroneal tendon. The method can also include imaging the inflamed peroneal tendon. The method can also include imaging the inflamed peroneal tendon after the ablating at least a portion of the inflamed peroneal tendon. The method can include comparing a measurement of the inflamed peroneal tendon before and after the ablating step. The method can include directing acoustical pressure or cavitation to the inflamed peroneal tendon after the ablating step further improving inflamed peroneal tendon. The method can include increasing blood perfusion to the ROI 115. The method can include administering a medicant to the ROI 115. The method can also include any of the steps of method 100.

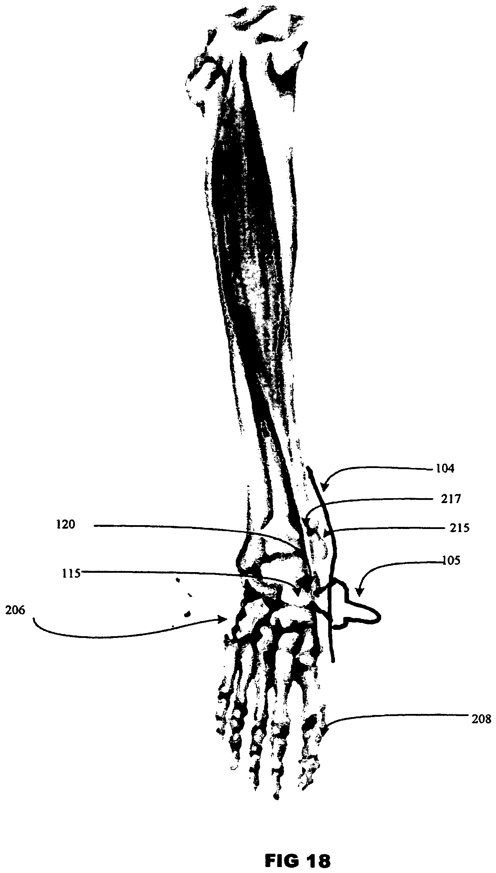

Referring to Figure 18, the tibialis anterior muscle 218 is the large muscle that runs down the outside of the shin. The tibialis anterior tendon 217 can be felt at the front of the ankle 215. Inflammation in the tibialis anterior tendon 217 can arise through overuse particularly through running on hard surfaces or in racket sports where you have to change direction frequently.

According to various embodiments, methods of treating inflamed tibialis anterior tendon are provided. Such a method can include targeting the inflamed tibialis anterior tendon in ROI 115, directing therapeutic ultrasound energy 120 to the inflamed tibialis anterior tendon, ablating at least a portion of the inflamed tibialis anterior tendon, and improving inflamed tibialis anterior tendon. The method can include coupling ultrasound probe 105 to ROI 115. The method can include focusing therapeutic ultrasound energy 120 to create a lesion 25 in a portion of the inflamed tibialis anterior tendon. The method can include creating a plurality of lesions 25 in the inflamed tibialis anterior tendon. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tibialis anterior tendon. The method can also include imaging the inflamed tibialis anterior tendon. The method can also include imaging the inflamed tibialis anterior tendon after the ablating at least a portion of the inflamed tibialis anterior tendon. The method can include comparing a measurement of the inflamed tibialis anterior tendon the fascia before and after the ablating step. The method can include directing acoustical pressure or cavitation to the inflamed tibialis anterior tendon after the ablating step further improving inflamed tibialis anterior tendon. The method can include increasing blood perfusion to the ROI 115. The method can include administering a medicant to the ROI 115. The method can also include any of the steps of method 100.

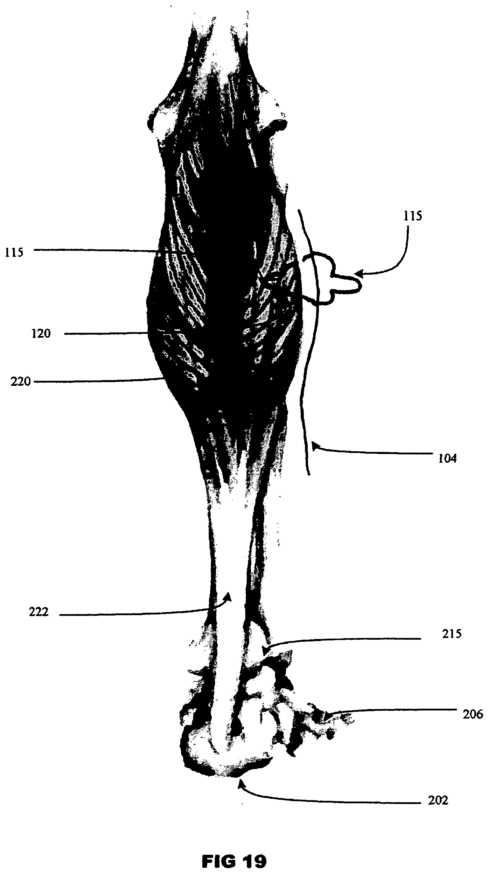

Turning to Figure 19, the calf muscles 202 consist of the Gastrocnemius which is the big muscle at the back of the lower leg and the Soleus muscle which is a smaller muscle lower down in the leg and under the Gastrocnemius. Gastrocnemius is the larger of the two muscles which attaches above the knee joint and inserts into the heel 202 via the Achilles tendon 222. The Soleus attaches below the knee joint and then also to the heel 202 via the Achilles tendon 222. Either of these two muscles can be strained (torn). Both muscles act to plantarflex the ankle. As gastrocnemius attaches above the knee it also helps with bending the knee. In this position, with the knee bent, soleus becomes the main plantarflexor. If the Soleus muscle is damaged might cause pain lower in the leg and also cause pain when contracting the muscle against resistance with the knee bent.

According to various embodiments, methods of treating tears, partial ruptures, or strains in the calf muscle 220 are provided. Such a method can include targeting the tear, partial rupture, or strain within the calf muscle 220 in ROI 115, directing therapeutic ultrasound energy 120 to the tear, partial rupture, or strain within the calf muscle 220, ablating at least a portion of the tear, partial rupture, or strain within the calf muscle 220, and improving the tear, partial rupture, or strain within the calf muscle 220. The method can include coupling ultrasound probe 105 to ROI 115. The method can include focusing therapeutic ultrasound energy 120 to create a lesion 25 in a portion of the tear, partial rupture, or strain within the calf muscle 220. The method can include creating a plurality of lesions 25 in the tear, partial rupture, or strain within the calf muscle 220. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear, partial rupture, or strain within the calf muscle 220. The method can also include imaging the tear, partial rupture, or strain within the calf muscle 220. The method can also include imaging the tear, partial rupture, or strain within the calf muscle 220 after the ablating at least a portion of the tear, partial rupture, or strain within the calf muscle 220. The method can include comparing a measurement of the tear, partial rupture, or strain within the calf muscle 220 before and after the ablating step. The method can include directing acoustical pressure or cavitation to the tear, partial rupture, or strain within the calf muscle 220 after the ablating step further improving the tear, partial rupture, or strain within the calf muscle 220. The method can include welding the tear or partial rupture within the calf muscle 220 with therapeutic ultrasound energy 120. The method can include increasing blood perfusion to the ROI 115. The method can include administering a medicant to the ROI 115. The method can also include any of the steps of method 100.

A sprained ankle or twisted ankle is a common cause of ankle pain. A sprain is stretching and or tearing of ligaments. The most common is an inversion sprain (or lateral ligament sprain) where the ankle turns over so the sole of the foot faces inwards, damaging the ligaments on the outside of the ankle.

According to various embodiments, methods of treating ligaments of the ankle 215 are provided. Such a method can include targeting the tear within a ligament of the ankle 215 in ROI 115, directing therapeutic ultrasound energy 120 to the tear within a ligament of the ankle 215, ablating at least a portion of the tear within a ligament of the ankle 215, and improving the tear within a ligament of the ankle 215. The method can include coupling ultrasound probe 105 to ROI 115. The method can include focusing therapeutic ultrasound energy 120 to create a lesion 25 in a portion of the tear within a ligament of the ankle 215. The method can include creating a plurality of lesions 25 in the tear within a ligament of the ankle 215. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear within a ligament of the ankle 215. The method can also include imaging the tear within a ligament of the ankle 215. The method can also include imaging the tear within a ligament of the ankle 215 after the ablating at least a portion of the tear within a ligament of the ankle 215. The method can include comparing a measurement of the tear within a ligament of the ankle 215 before and after the ablating step. The method can include directing acoustical pressure or cavitation to the tear within a ligament of the ankle 215 after the ablating step further improving the tear within a ligament of the ankle 215. The method can include welding the tear within a ligament of the ankle 215 with therapeutic ultrasound energy 120. The method can include increasing blood perfusion to the ROI 115. The method can include administering a medicant to the ROI 115. The method can also include any of the steps of method 100.

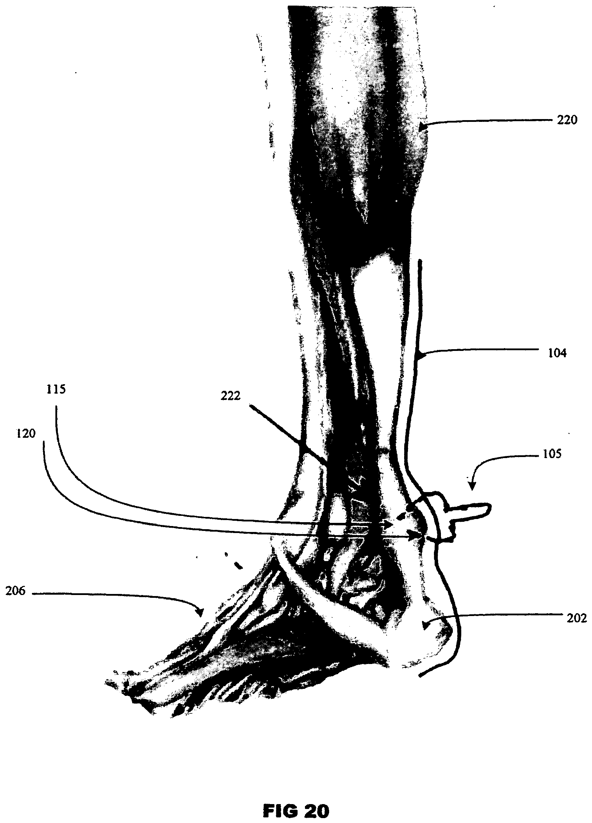

Referring to Figure 20, Achilles tendonitis is often now being referred to as Achilles tendinopathy. On investigation, the main finding is usually degenerated tissue with a loss of normal fiber structure. Achilles tendonitis can be either acute or chronic. The condition can also be either at the attachment point to the heel 202 or in the mid-portion of the tendon 222, typically around 4cm above the heel 202. Healing of the Achilles tendon 222 is often slow, due to its poor blood supply.

According to various embodiments, methods of treating a strained Achilles tendon 222 are provided. Such a method can include targeting degenerated tissue within the Achilles tendon 222 in ROI 115, directing therapeutic ultrasound energy 120 to the degenerated tissue within the Achilles tendon 222, ablating at least a portion of the degenerated tissue within the Achilles tendon 222, and improving the degenerated tissue within the Achilles tendon 222. The method can include coupling ultrasound probe 105 to ROI 115. The method can include focusing therapeutic ultrasound energy 120 to create a lesion 25 in a portion of the degenerated tissue within the Achilles tendon 222. The method can include creating a plurality of lesions 25 in the degenerated tissue within the Achilles tendon 222. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the degenerated tissue within the Achilles tendon 222. The method can also include imaging degenerated tissue within the Achilles tendon 222. The method can also include imaging the degenerated tissue within the Achilles tendon 222 after the ablating at least a portion of the degenerated tissue within the Achilles tendon 222. The method can include comparing a measurement of the degenerated tissue within the Achilles tendon 222 before and after the ablating step. The method can include directing acoustical pressure or cavitation to the degenerated tissue within the Achilles tendon 222 after the ablating step further improving the degenerated tissue within the Achilles tendon 222. The method can include welding the degenerated tissue within the Achilles tendon 222 with therapeutic ultrasound energy 120. The method can include increasing blood flow to the degenerated tissue within the Achilles tendon 222. The method can include increasing blood perfusion to the ROI 115. The method can include administering a medicant to the ROI 115. The method can also include any of the steps of method 100.

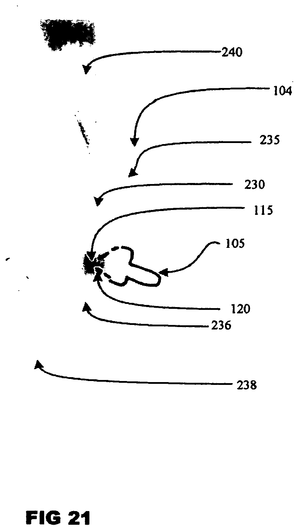

With reference to Figure 21, the patella tendon 236 joins the patella 230 to the shin bone or tibia 238. This tendon 236 is extremely strong and allows the quadriceps muscle 240 group to straighten the leg. The quadriceps 240 actively straighten the knee 235 in jumping to propel an individual off the ground as well as functioning in stabilizing their landing. As such this tendon comes under a large amount of stress especially in individuals who actively put extra strain on the knee joint 235 such as those who regularly perform sports that involve direction changing and jumping movements. With repeated strain, micro-tears as well as collagen degeneration may occur as a result in the tendon 236. This is known as patellar tendinopathy or Jumpers Knee.

According to various embodiments, methods of treating jumper's knee are provided. Such a method can include targeting degenerated tissue within the patella tendon 236 in ROI 115, directing therapeutic ultrasound energy 120 to the degenerated tissue or micro-tears within the patella tendon 236, ablating at least a portion of the degenerated tissue or micro-tears within the patella tendon 236, and improving the degenerated tissue or micro-tears within the patella tendon 236. The method can include coupling ultrasound probe 105 to ROI 115. The method can include focusing therapeutic ultrasound energy 120 to create a lesion 25 in a portion of the degenerated tissue or micro-tears within the patella tendon 236. The method can include creating a plurality of lesions 25 in the degenerated tissue or micro-tears within the patella tendon 236. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the degenerated tissue or micro-tears within the patella tendon 236. The method can also include imaging the degenerated tissue or micro-tears within the patella tendon 236. The method can also include imaging the degenerated tissue or micro-tears within the patella tendon 236 after the ablating at least a portion of the degenerated tissue or micro-tears within the patella tendon 236. The method can include comparing a measurement of the degenerated tissue or micro-tears within the patella tendon 236 before and after the ablating step. The method can include directing acoustical pressure or cavitation to the degenerated tissue or micro-tears within the patella tendon 236 after the ablating step further improving the degenerated tissue or micro-tears within the patella tendon 236. The method can include welding the degenerated tissue or micro-tears within the patella tendon 236 with therapeutic ultrasound energy 120. The method can include increasing collagen in the degenerated tissue or micro-tears within the patella tendon 236. The method can include increasing blood perfusion to the ROI 115. The method can include administering a medicant to the ROI 115. The method can also include any of the steps of method 100.

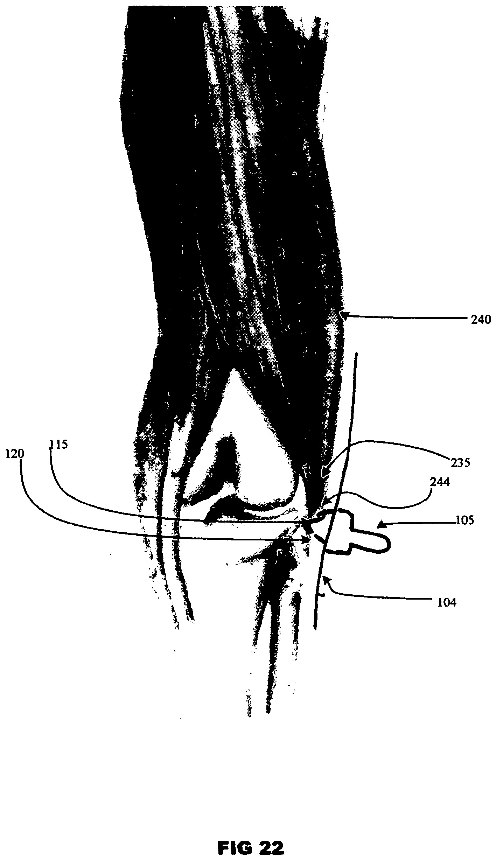

Turning to Figure 22, the hamstring muscles 240 consist of the Biceps femoris, Semitendinosus and Semimembranosus. The muscles insert at the back of the knee 235 through hamstring tendons 244. It is possible for these tendons 244 to be torn, partial ruptured, or strained during an explosive or kicking movement. In addition the hamstring muscles 240 can be torn or strained.

According to various embodiments, methods of treating tears, partial ruptures, or strains in a hamstring tendon 244 are provided. Such a method can include targeting the tear, partial rupture, or strain within a hamstring tendon 244 in ROI 115, directing therapeutic ultrasound energy 120 to the tear, partial rupture, or strain within a hamstring tendon 244, ablating at least a portion of the tear, partial rupture, or strain within a hamstring tendon 244, and improving the tear, partial rupture, or strain within a hamstring tendon 244. The method can include coupling ultrasound probe 105 to ROI 115. The method can include focusing therapeutic ultrasound energy 120 to create a lesion 25 in a portion of the tear, partial rupture, or strain within a hamstring tendon 244. The method can include creating a plurality of lesions 25 in the tear, partial rupture, or strain within a hamstring tendon 244. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear, partial rupture, or strain within a hamstring tendon 244. The method can also include imaging the tear, partial rupture, or strain within a hamstring tendon 244. The method can also include imaging the tear, partial rupture, or strain within a hamstring tendon 244 after the ablating at least a portion of the tear, partial rupture, or strain within a hamstring tendon 244. The method can include comparing a measurement of the tear, partial rupture, or strain within a hamstring tendon 244 before and after the ablating step. The method can include directing acoustical pressure or cavitation to the tear, partial rupture, or strain within a hamstring tendon 244 after the ablating step further improving the tear, partial rupture, or strain within a hamstring tendon 244. The method can include welding the tear or partial rupture within a hamstring tendon 244 with therapeutic ultrasound energy 120. The method can include increasing blood perfusion to the ROI 115. The method can include administering a medicant to the ROI 115. The method can also include any of the steps of method 100.

According to various embodiments, methods of treating tears, partial ruptures, or strains in a hamstring muscle 240 are provided. Such a method can include targeting the tear, partial rupture, or strain within a hamstring muscle 240 in ROI 115, directing therapeutic ultrasound energy 120 to the tear, partial rupture, or strain within a hamstring muscle 240, ablating at least a portion of the tear, partial rupture, or strain within a hamstring muscle 240, and improving the tear, partial rupture, or strain within a hamstring muscle 240. The method can include coupling ultrasound probe 105 to ROI 115. The method can include focusing therapeutic ultrasound energy 120 to create a lesion 25 in a portion of the tear, partial rupture, or strain within a hamstring muscle 240. The method can include creating a plurality of lesions 25 in the tear, partial rupture, or strain within a hamstring muscle 240. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear, partial rupture, or strain within a hamstring muscle 240. The method can also include imaging the tear, partial rupture, or strain within a hamstring muscle 240. The method can also include imaging the tear, partial rupture, or strain within a hamstring muscle 240 after the ablating at least a portion of the tear, partial rupture, or strain within a hamstring muscle 240. The method can include comparing a measurement of the tear, partial rupture, or strain within a hamstring muscle 240 before and after the ablating step. The method can include directing acoustical pressure or cavitation to the tear, partial rupture, or strain within a hamstring muscle 240 after the ablating step further improving the tear, partial rupture, or strain within a hamstring muscle 240. The method can include welding the tear or partial rupture within a hamstring muscle 240 with therapeutic ultrasound energy 120. The method can include increasing blood perfusion to the ROI 115. The method can include administering a medicant to the ROI 115. The method can also include any of the steps of method 100.

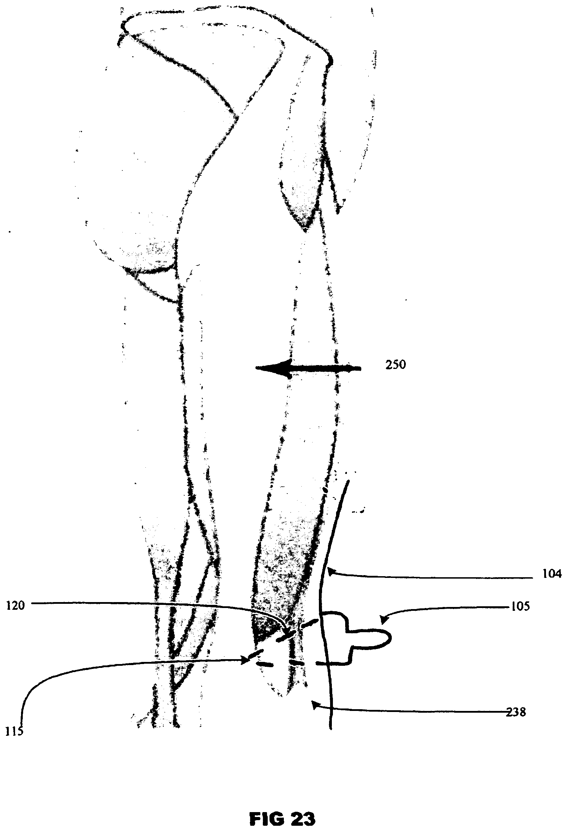

Turning now to Figure 23, Iliotibial band syndrome is often referred to by other names, most commonly, runner's knee and Iliotibial band friction syndrome. The Iliotibial band 250 is a sheath of thick, fibrous connective tissue which attaches at the top to both the iliac crest (hip bone) and the Tensor fascia latae muscle. It then runs down the outside of the thigh and inserts into the outer surface of the Tibia 238. Its purpose is to extend the knee joint 235 as well as to abduct the hip. As the Iliotibial band 250 passes over the lateral epicondyle of the femur it is prone to friction. At an angle of approximately 20-30 degrees the Iliotibial band 250 flicks across the lateral epicondyle. When the knee 235 is being straightened it flicks in front of the epicondyle and when it is bent, it flicks back behind.

According to various embodiments, methods of treating inflamed portions of the Iliotibial band 250 are provided. Such a method can include targeting an inflamed portion of the Iliotibial band 250 in ROI 115, directing therapeutic ultrasound energy 120 to an inflamed portion of the Iliotibial band 250, ablating at least a portion of an inflamed portion of the Iliotibial band 250, and improving an inflamed portion of the Iliotibial band 250. The method can include coupling ultrasound probe 105 to ROI 115. The method can include focusing therapeutic ultrasound energy 120 to create a lesion 25 in a portion of an inflamed portion of the Iliotibial band 250. The method can include creating a plurality of lesions 25 in an inflamed portion of the Iliotibial band 250. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to an inflamed portion of the Iliotibial band 250. The method can also include imaging an inflamed portion of the Iliotibial band 250. The method can also include imaging an inflamed portion of the Iliotibial band 250 after the ablating at least a portion of an inflamed portion of the Iliotibial band 250. The method can include comparing a measurement of an inflamed portion of the Iliotibial band 250 before and after the ablating step. The method can include directing acoustical pressure or cavitation to an inflamed portion of the Iliotibial band 250 after the ablating step further improving an inflamed portion of the Iliotibial band 250. The method can include increasing blood perfusion to the ROI 115. The method can include administering a medicant to the ROI 115. The method can also include any of the steps of method 100.

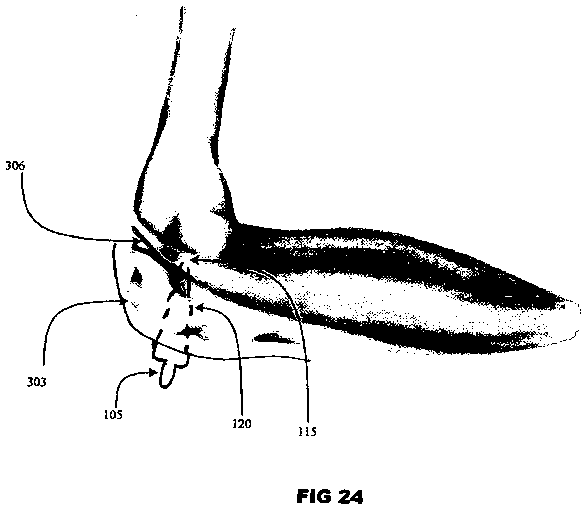

Turning to Figure 24, tennis elbow or lateral epicondylitis is an extremely common injury that originally got its name because it is a frequent tennis injury, appearing in a large proportion of tennis players. Nevertheless it commonly manifests in a vast proportion of people who do not play tennis at all. Lateral epicondylitis occurs most commonly in the tendon 306 of the extensor carpi radialis brevis muscle at approximately 2cm below the outer edge of the elbow joint 303 or lateral epicondyle of the humerus bone. Specific inflammation is rarely present in the tendon but there is an increase in pain receptors in the area making the region extremely tender.

According to various embodiments, methods of treating tennis elbow are provided. Such a method can include targeting inflamed tissue near or at the tendon 306 of the extensor carpi radialis brevis musclein in ROI 115, directing therapeutic ultrasound energy 120 to the inflamed tissue near or at the tendon 306 of the extensor carpi radialis brevis musclein, ablating at least a portion of the inflamed tissue near or at the tendon 306 of the extensor carpi radialis brevis musclein, and improving the inflamed tissue near or at the tendon 306 of the extensor carpi radialis brevis musclein. The method can include coupling ultrasound probe 105 to ROI 115. The method can include focusing therapeutic ultrasound energy 120 to create a lesion 25 in a portion of the inflamed tissue near or at the tendon 306 of the extensor carpi radialis brevis musclein. The method can include creating a plurality of lesions 25 in the inflamed tissue near or at the tendon 306 of the extensor carpi radialis brevis musclein. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at the tendon 306 of the extensor carpi radialis brevis musclein. The method can also include imaging an inflamed portion of the at the tendon 306 of the extensor carpi radialis brevis musclein. The method can also include imaging the inflamed tissue near or at the tendon 306 of the extensor carpi radialis brevis musclein after the ablating at least a portion of the inflamed tissue near or at the tendon 306 of the extensor carpi radialis brevis musclein. The method can include comparing a measurement of the inflamed tissue near or at the tendon 306 of the extensor carpi radialis brevis musclein before and after the ablating step. The method can include directing acoustical pressure or cavitation to the inflamed tissue near or at the tendon 306 of the extensor carpi radialis brevis musclein after the ablating step further improving the inflamed tissue near or at the tendon 306 of the extensor carpi radialis brevis musclein. The method can include increasing blood perfusion to the ROI 115. The method can include administering a medicant to the ROI 115. The method can also include any of the steps of method 100.

According to various embodiments, methods of treating tennis elbow are provided. Such a method can include targeting micro-tears within tendon 306 in ROI 115, directing therapeutic ultrasound energy 120 to the micro-tears within tendon 306, ablating at least a portion of the micro-tears within tendon 306, and improving the micro-tears within tendon 306. The method can include coupling ultrasound probe 105 to ROI 115. The method can include focusing therapeutic ultrasound energy 120 to create a lesion 25 in a portion of the micro-tears within tendon 306. The method can include creating a plurality of lesions 25 in the tendon 306. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within tendon 306. The method can also include imaging micro-tears within tendon 306. The method can also include imaging micro-tears within tendon 306 after the ablating micro-tears within tendon 306. The method can include comparing a measurement of the micro-tears within tendon 306 before and after the ablating step. The method can include directing acoustical pressure or cavitation to the micro-tears within tendon 306 after the ablating step further improving the micro-tears within tendon 306. The method can include welding the micro-tears within tendon 306 with therapeutic ultrasound energy 120. The method can include increasing blood perfusion to the ROI 115. The method can include administering a medicant to the ROI 115. The method can also include any of the steps of method 100.

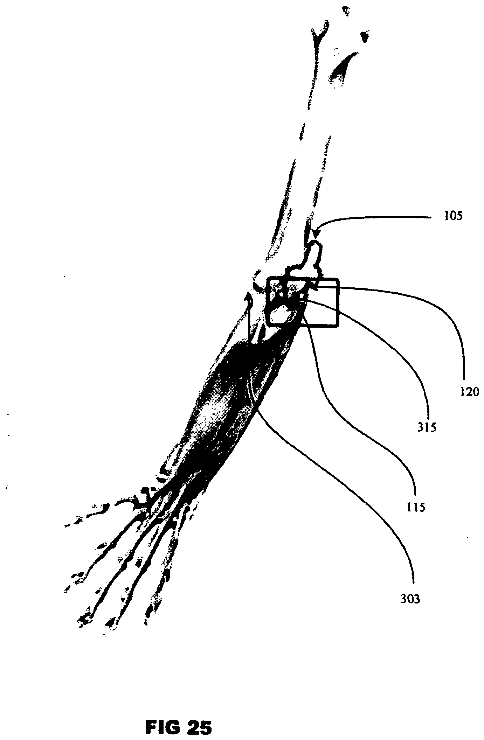

With reference to Figure 25, golfer's elbow is a similar injury to tennis elbow only it affects the inside of the elbow instead. Golfer's elbow is more common in throwers and golfers hence the 'nicknames'. Also known as flexor / pronator tendinopathy this elbow pain is seen in tennis players who use a lot of top spin on their forehand shots.

According to various embodiments, methods of treating golfer's elbow are provided. Such a method can include targeting inflamed tissue near or at common flexor tendon 315 in ROI 115, directing therapeutic ultrasound energy 120 to the inflamed tissue near or at common flexor tendon 315, ablating at least a portion of the inflamed tissue near or at common flexor tendon 315, and improving the inflamed tissue near or at common flexor tendon 315. The method can include coupling ultrasound probe 105 to ROI 115. The method can include focusing therapeutic ultrasound energy 120 to create a lesion 25 in a portion of the inflamed tissue near or at common flexor tendon 315. The method can include creating a plurality of lesions 25 in the inflamed tissue near or at common flexor tendon 315. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at common flexor tendon 315. The method can also include imaging an inflamed portion of common flexor tendon 315. The method can also include imaging the inflamed tissue near or at common flexor tendon 315 after the ablating at least a portion of the inflamed tissue near or at common flexor tendon 315. The method can include comparing a measurement of the inflamed tissue near or at common flexor tendon 315 before and after the ablating step. The method can include directing acoustical pressure or cavitation to the inflamed tissue near or at common flexor tendon 315 after the ablating step further improving the inflamed tissue near or at common flexor tendon 315. The method can include increasing blood perfusion to the ROI 115. The method can include administering a medicant to the ROI 115. The method can also include any of the steps of method 100.

According to various embodiments, methods of treating golfer's elbow are provided. Such a method can include targeting micro-tears within common flexor tendon 315 in ROI 115, directing therapeutic ultrasound energy 120 to the micro-tears within common flexor tendon 315, ablating at least a portion of the micro-tears within common flexor tendon 315, and improving the micro-tears within common flexor tendon 315. The method can include coupling ultrasound probe 105 to ROI 115. The method can include focusing therapeutic ultrasound energy 120 to create a lesion 25 in a portion of the micro-tears within common flexor tendon 315. The method can include creating a plurality of lesions 25 in the micro-tears within common flexor tendon 315. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within common flexor tendon 315. The method can also include imaging micro-tears within common flexor tendon 315. The method can also include imaging micro-tears within common flexor tendon 315 after the ablating at least a portion of the micro-tears within common flexor tendon 315. The method can include comparing a measurement of the micro-tears within common flexor tendon 315 before and after the ablating step. The method can include directing acoustical pressure or cavitation to the micro-tears within common flexor tendon 315 after the ablating step further improving the micro-tears within common flexor tendon 315. The method can include welding the micro-tears within common flexor tendon 315 with therapeutic ultrasound energy 120. The method can include increasing blood perfusion to the ROI 115. The method can include administering a medicant to the ROI 115. The method can also include any of the steps of method 100.

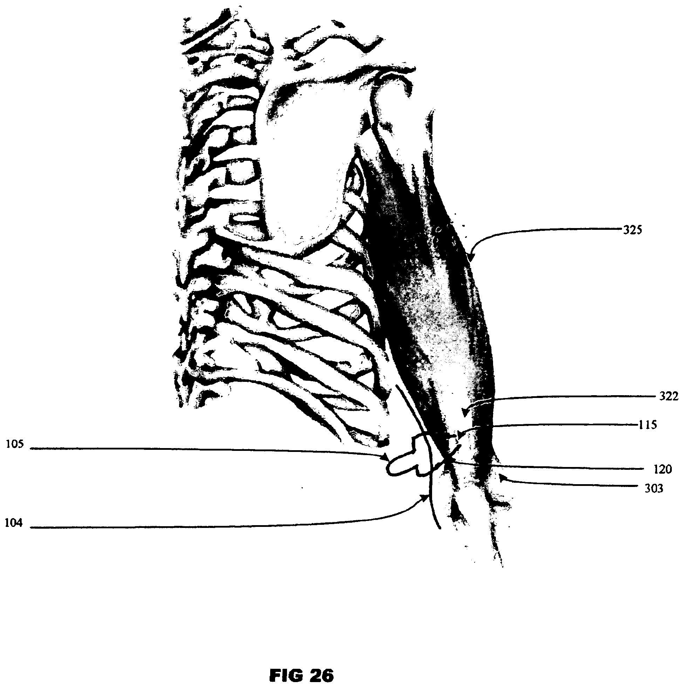

With reference to Figure 26, the triceps tendon 322 connects the triceps muscle 325 into the back of the elbow 303. A fall onto one's hands can partial rupture the triceps tendon 322. Over-doing weights or trying to push something too heavy can also partial rupture the triceps tendon 322. Alternatively, the triceps tendon 322 can become inflamed through over use.

According to various embodiments, methods of treating inflamed tissue near or at the triceps tendon 322 are provided. Such a method can include targeting inflamed tissue near or at the triceps tendon 322 in ROI 115, directing therapeutic ultrasound energy 120 to the inflamed tissue near or at the triceps tendon 322, ablating at least a portion of the inflamed tissue near or at the triceps tendon 322, and improving the inflamed tissue near or at the triceps tendon 322. The method can include coupling ultrasound probe 105 to ROI 115. The method can include focusing therapeutic ultrasound energy 120 to create a lesion 25 in a portion of the inflamed tissue near or at the triceps tendon 322. The method can include creating a plurality of lesions 25 in the inflamed tissue near or at the triceps tendon 322. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at the triceps tendon 322. The method can also include imaging an inflamed portion of the triceps tendon 322. The method can also include imaging the inflamed tissue near or at the triceps tendon 322 after the ablating at least a portion of the inflamed tissue near or at the triceps tendon 322. The method can include comparing a measurement of the inflamed tissue near or at the triceps tendon 322 before and after the ablating step. The method can include directing acoustical pressure or cavitation to the inflamed tissue near or at the triceps tendon 322 after the ablating step further improving the inflamed tissue near or at the triceps tendon 322. The method can include increasing blood perfusion to the ROI 115. The method can include administering a medicant to the ROI 115. The method can also include any of the steps of method 100.

According to various embodiments, methods of treating tendonitis in the triceps tendon 322 are provided. Such a method can include targeting micro-tears within the triceps tendon 322 in ROI 115, directing therapeutic ultrasound energy 120 to the micro-tears within the triceps tendon 322, ablating at least a portion of the micro-tears within the triceps tendon 322, and improving the micro-tears within the triceps tendon 322. The method can include coupling ultrasound probe 105 to ROI 115. The method can include focusing therapeutic ultrasound energy 120 to create a lesion 25 in a portion of the micro-tears within the triceps tendon 322. The method can include creating a plurality of lesions 25 in the micro-tears within the triceps tendon 322. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within the triceps tendon 322. The method can also include imaging micro-tears within the triceps tendon 322. The method can also include imaging micro-tears within the triceps tendon 322 after the ablating at least a portion of the micro-tears within the triceps tendon 322. The method can include comparing a measurement of the micro-tears within the triceps tendon 322 before and after the ablating step. The method can include directing acoustical pressure or cavitation to the micro-tears within the triceps tendon 322 after the ablating step further improving the micro-tears within the triceps tendon 322. The method can include welding the micro-tears within the triceps tendon 322 with therapeutic ultrasound energy 120. The method can include increasing blood perfusion to the ROI 115. The method can include administering a medicant to the ROI 115. The method can also include any of the steps of method 100.

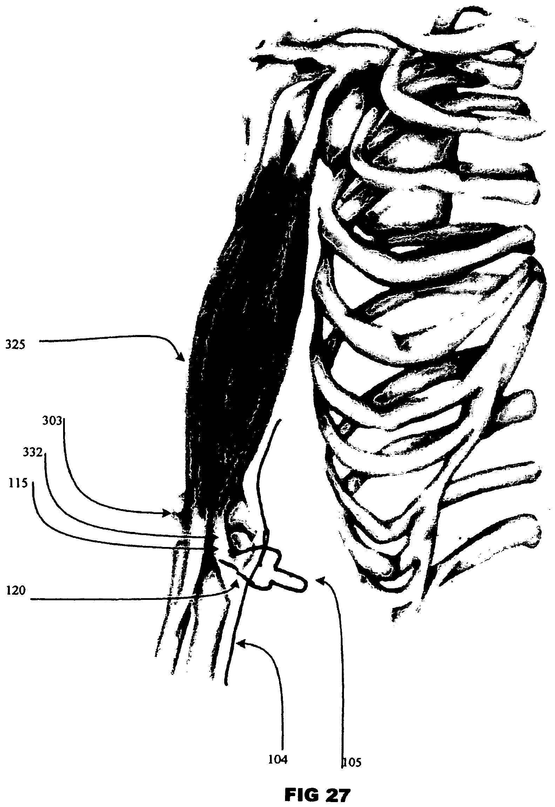

With reference to Figure 27, the biceps tendon 332 connects the biceps muscle 325 into the elbow 303. An injury to the biceps tendon 332 is more likely to affect weight lifters who over do the biceps curls. People who do a lot of writing such as students can also be prone to this injury due to the biceps tendon 332 inserts into the inside of the elbow 303 and can become inflamed if overused.

According to various embodiments, methods of treating inflamed tissue near or at the biceps tendon 332 are provided. Such a method can include targeting inflamed tissue near or at the biceps tendon 332 in ROI 115, directing therapeutic ultrasound energy 120 to the inflamed tissue near or at the biceps tendon 332, ablating at least a portion of the inflamed tissue near or at the biceps tendon 332, and improving the inflamed tissue near or at the biceps tendon 332. The method can include coupling ultrasound probe 105 to ROI 115. The method can include focusing therapeutic ultrasound energy 120 to create a lesion 25 in a portion of the inflamed tissue near or at the biceps tendon 332. The method can include creating a plurality of lesions 25 in the inflamed tissue near or at the biceps tendon 332. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at the biceps tendon 332. The method can also include imaging an inflamed portion of the biceps tendon 332. The method can also include imaging the inflamed tissue near or at the biceps tendon 332 after the ablating at least a portion of the inflamed tissue near or at the biceps tendon 332. The method can include comparing a measurement of the inflamed tissue near or at the biceps tendon 332 before and after the ablating step. The method can include directing acoustical pressure or cavitation to the inflamed tissue near or at the biceps tendon 332 after the ablating step further improving the inflamed tissue near or at the biceps tendon 332. The method can include increasing blood perfusion to the ROI 115. The method can include administering a medicant to the ROI 115. The method can also include any of the steps of method 100.

According to various embodiments, methods of treating tendonitis in the biceps tendon 332 are provided. Such a method can include targeting micro-tears within the biceps tendon 332 in ROI 115, directing therapeutic ultrasound energy 120 to the micro-tears within the biceps tendon 332, ablating at least a portion of the micro-tears within the biceps tendon 332, and improving the micro-tears within the biceps tendon 332. The method can include coupling ultrasound probe 105 to ROI 115. The method can include focusing therapeutic ultrasound energy 120 to create a lesion 25 in a portion of the micro-tears within the biceps tendon 332. The method can include creating a plurality of lesions 25 in the micro-tears within the biceps tendon 332. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within the biceps tendon 332. The method can also include imaging micro-tears within the biceps tendon 332. The method can also include imaging micro-tears within the biceps tendon 332 after the ablating at least a portion of the micro-tears within the biceps tendon 332. The method can include comparing a measurement of the micro-tears within the biceps tendon 332 before and after the ablating step. The method can include directing acoustical pressure or cavitation to the micro-tears within the biceps tendon 332 after the ablating step further improving the micro-tears within the biceps tendon 332. The method can include welding the micro-tears within the biceps tendon 332 with therapeutic ultrasound energy 120. The method can include increasing blood perfusion to the ROI 115. The method can include administering a medicant to the ROI 115. The method can also include any of the steps of method 100.

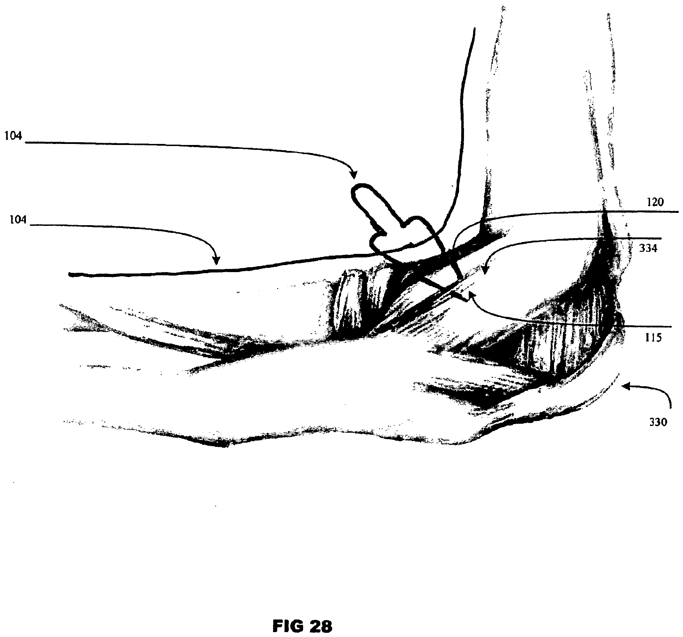

With attention turned to Figure 28, the medial elbow ligaments 334 are situated on the inside of the elbow 303 and help provide stability to the joint. Damage to these ligaments can occur through impact injury or accident or from repetitive overuse for example throwing with poor technique. For example Javelin throwers who throw with a low elbow 303. A sprain is stretching and or tearing of a ligament of the elbow 303.

According to various embodiments, methods of treating ligaments of the elbow 330 are provided. Such a method can include targeting the tear within a ligament of the elbow 330 in ROI 115, directing therapeutic ultrasound energy 120 to the tear within a ligament of the elbow 330, ablating at least a portion of the tear within a ligament of the elbow 330, and improving the tear within a ligament of the elbow 330. The method can include coupling ultrasound probe 105 to ROI 115. The method can include focusing therapeutic ultrasound energy 120 to create a lesion 25 in a portion of the tear within a ligament of the elbow 330. The method can include creating a plurality of lesions 25 in the tear within a ligament of the elbow 330. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear within a ligament of the elbow 330. The method can also include imaging the tear within a ligament of the elbow 330. The method can also include imaging the tear within a ligament of the elbow 330 after the ablating at least a portion of the tear within a ligament of the elbow 330. The method can include comparing a measurement of the tear within a ligament of the elbow 330 before and after the ablating step. The method can include directing acoustical pressure or cavitation to the tear within a ligament of the elbow 330 after the ablating step further improving the tear within a ligament of the elbow 330. The method can include welding the tear within a ligament of the elbow 330 with therapeutic ultrasound energy 120. The method can include increasing blood perfusion to the ROI 115. The method can include administering a medicant to the ROI 115. The method can also include any of the steps of method 100.

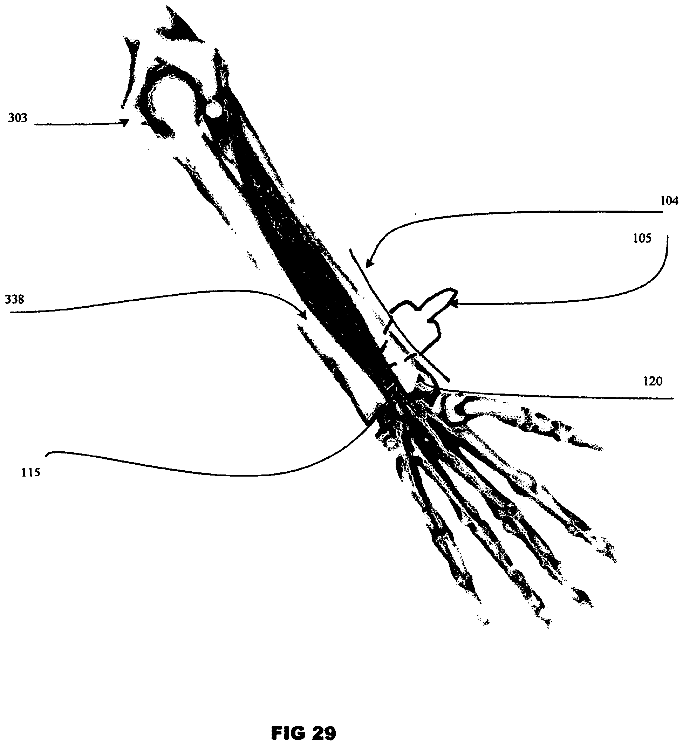

With reference to Figure 29, as a result of overuse the muscles and tendons in the forearm 338 can become inflamed. The injury is particularly common in rowers and canoeists but can also affect racket sport players.

According to various embodiments, methods of treating inflamed tissue near or at a tendon in the forearm 338 are provided. Such a method can include targeting inflamed tissue near or at a tendon in the forearm 338 in ROI 115, directing therapeutic ultrasound energy 120 to the inflamed tissue near or at a tendon in the forearm 338, ablating at least a portion of the inflamed tissue near or at a tendon in the forearm 338, and improving the inflamed tissue near or at a tendon in the forearm 338. The method can include coupling ultrasound probe 105 to ROI 115. The method can include focusing therapeutic ultrasound energy 120 to create a lesion 25 in a portion of the inflamed tissue near or at a tendon in the forearm 338. The method can include creating a plurality of lesions 25 in the inflamed tissue near or at a tendon in the forearm 338. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at a tendon in the forearm 338. The method can also include imaging an inflamed portion of a tendon in the forearm 338. The method can also include imaging the inflamed tissue near or at a tendon in the forearm 338 after the ablating at least a portion of the inflamed tissue near or at a tendon in the forearm 338. The method can include comparing a measurement of the inflamed tissue near or at a tendon in the forearm 338 before and after the ablating step. The method can include directing acoustical pressure or cavitation to the inflamed tissue near or at a tendon in the forearm 338 after the ablating step further improving the inflamed tissue near or at a tendon in the forearm 338. The method can include increasing blood perfusion to the ROI 115. The method can include administering a medicant to the ROI 115. The method can also include any of the steps of method 100.

According to various embodiments, methods of treating tendonitis in a tendon in the forearm 338 are provided. Such a method can include targeting micro-tears within a tendon in the forearm 338 in ROI 115, directing therapeutic ultrasound energy 120 to the micro-tears within a tendon in the forearm 338, ablating at least a portion of the micro-tears within a tendon in the forearm 338, and improving the micro-tears within a tendon in the forearm 338. The method can include coupling ultrasound probe 105 to ROI 115. The method can include focusing therapeutic ultrasound energy 120 to create a lesion 25 in a portion of the micro-tears within a tendon in the forearm 338. The method can include creating a plurality of lesions 25 in the micro-tears within a tendon in the forearm 338. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within a tendon in the forearm 338. The method can also include imaging micro-tears within a tendon in the forearm 338. The method can also include imaging micro-tears within a tendon in the forearm 338 after the ablating at least a portion of the micro-tears within a tendon in the forearm 338. The method can include comparing a measurement of the micro-tears within a tendon in the forearm 338 before and after the ablating step. The method can include directing acoustical pressure or cavitation to the micro-tears within a tendon in the forearm 338 after the ablating step further improving the micro-tears within a tendon in the forearm 338. The method can include welding the micro-tears within a tendon in the forearm 338 with therapeutic ultrasound energy 120. The method can include increasing blood perfusion to the ROI 115. The method can include administering a medicant to the ROI 115. The method can also include any of the steps of method 100.

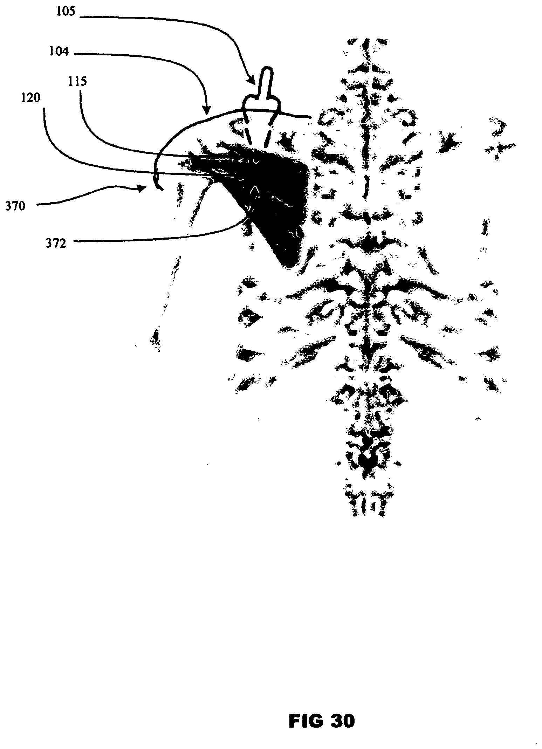

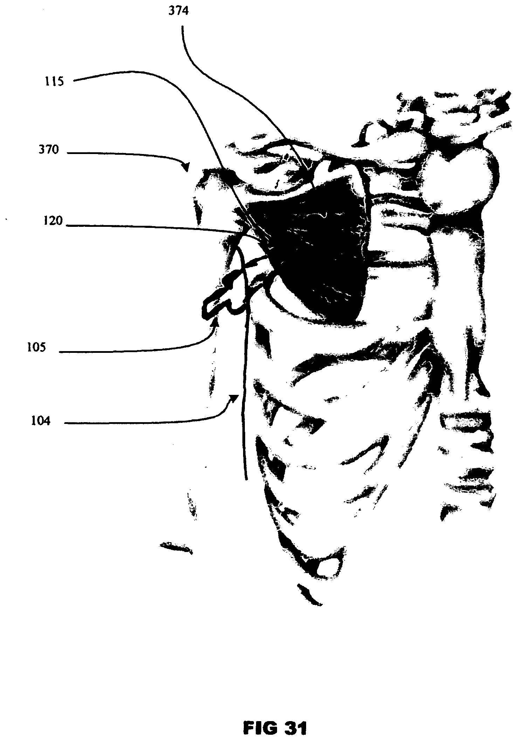

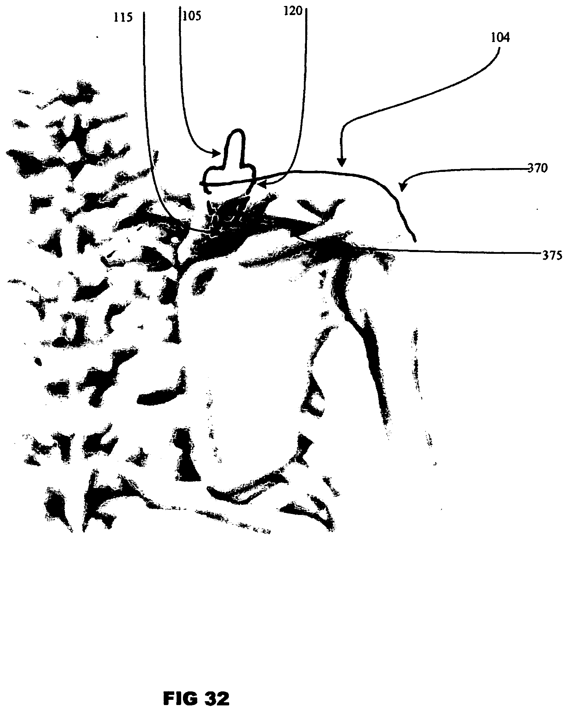

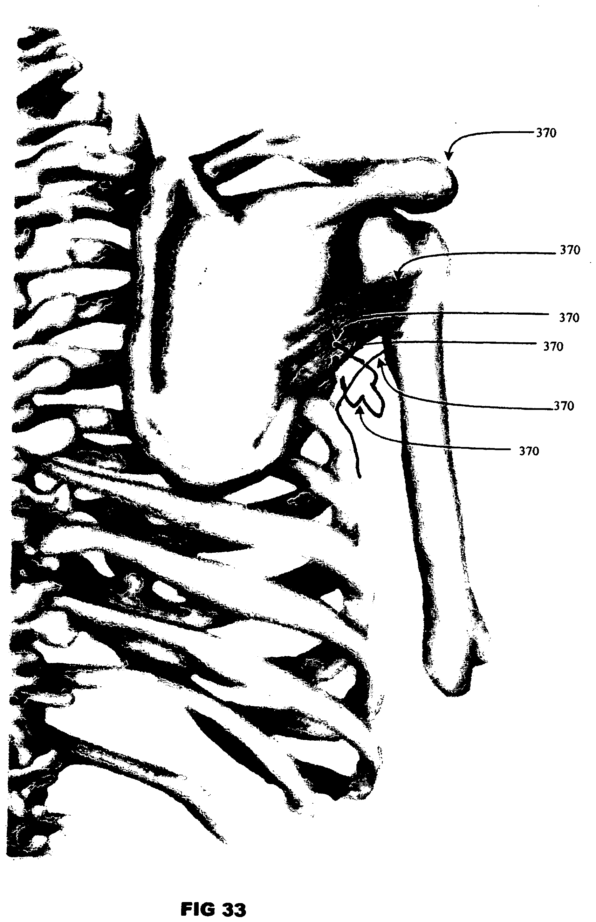

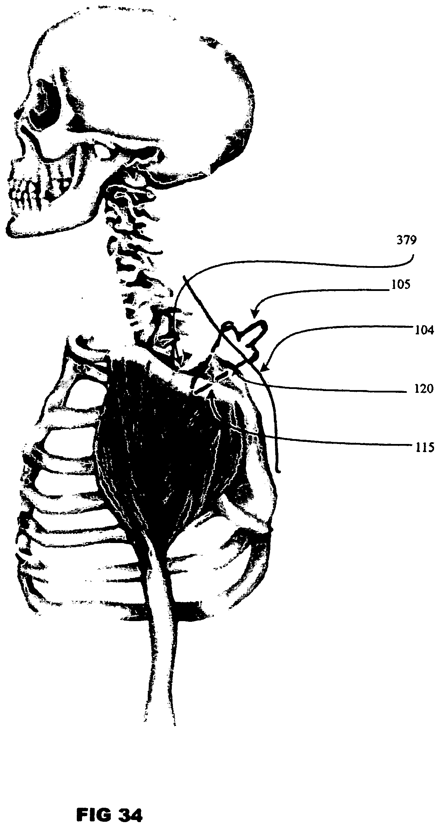

With reference to Figures 30-34, muscle of the shoulder 370 can be a torn, partial ruptured, or strained. For example, the rotator cuff muscles, such as Supraspinatus 375 and Infraspinatus 372 can be torn, partial ruptured or strained by throwing motions or by swimming. In addition, the deltoid muscle 379 can be torn, partial ruptured or strained by participation in sports or by heavy lifting.

According to various embodiments, methods of treating tears, partial ruptures, or strains in a muscle of the shoulder 370 are provided. Such a method can include targeting the tear, partial rupture, or strain within a muscle of the shoulder 370 in ROI 115, directing therapeutic ultrasound energy 120 to the tear, partial rupture, or strain within a muscle of the shoulder 370, ablating at least a portion of the tear, partial rupture, or strain within a muscle of the shoulder 370, and improving the tear, partial rupture, or strain within a muscle of the shoulder 370. The method can include coupling ultrasound probe 105 to ROI 115. The method can include focusing therapeutic ultrasound energy 120 to create a lesion 25 in a portion of the tear, partial rupture, or strain within a muscle of the shoulder 370. The method can include creating a plurality of lesions 25 in the tear, partial rupture, or strain within a muscle of the shoulder 370. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the tear, partial rupture, or strain within a muscle of the shoulder 370. The method can also include imaging the tear, partial rupture, or strain within a muscle of the shoulder 370. The method can also include imaging the tear, partial rupture, or strain within a muscle of the shoulder 370 after the ablating at least a portion of the tear, partial rupture, or strain within a muscle of the shoulder 370. The method can include comparing a measurement of the tear, partial rupture, or strain within a muscle of the shoulder 370 before and after the ablating step. The method can include directing acoustical pressure or cavitation to the tear, partial rupture, or strain within a muscle of the shoulder 370 after the ablating step further improving the tear, partial rupture, or strain within a muscle of the shoulder 370. The method can include welding the tear or partial rupture within a muscle of the shoulder 370 with therapeutic ultrasound energy 120. The method can include increasing blood perfusion to the ROI 115. The method can include administering a medicant to the ROI 115. The method can also include any of the steps of method 100.

According to various embodiments, methods of treating inflamed tissue near or at a tendon in the shoulder 370 are provided. Such a method can include targeting inflamed tissue near or at a tendon in the shoulder 370 in ROI 115, directing therapeutic ultrasound energy 120 to the inflamed tissue near or at a tendon in the shoulder 370, ablating at least a portion of the inflamed tissue near or at a tendon in the shoulder 370, and improving the inflamed tissue near or at a tendon in the shoulder 370. The method can include coupling ultrasound probe 105 to ROI 115. The method can include focusing therapeutic ultrasound energy 120 to create a lesion 25 in a portion of the inflamed tissue near or at a tendon in the shoulder 370. The method can include creating a plurality of lesions 25 in the inflamed tissue near or at a tendon in the shoulder 370. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at a tendon in the shoulder 370. The method can also include imaging an inflamed portion of a tendon in the shoulder 370. The method can also include imaging the inflamed tissue near or at a tendon in the shoulder 370 after the ablating at least a portion of the inflamed tissue near or at a tendon in the shoulder 370. The method can include comparing a measurement of the inflamed tissue near or at a tendon in the shoulder 370 before and after the ablating step. The method can include directing acoustical pressure or cavitation to the inflamed tissue near or at a tendon in the shoulder 370 after the ablating step further improving the inflamed tissue near or at a tendon in the shoulder 370. The method can include increasing blood perfusion to the ROI 115. The method can include administering a medicant to the ROI 115. The method can also include any of the steps of method 100.

According to various embodiments, methods of treating tendonitis in a tendon in the shoulder 370 are provided. Such a method can include targeting micro-tears within a tendon in the shoulder 370 in ROI 115, directing therapeutic ultrasound energy 120 to the micro-tears within a tendon in the shoulder 370, ablating at least a portion of the micro-tears within a tendon in the shoulder 370, and improving the micro-tears within a tendon in the shoulder 370. The method can include coupling ultrasound probe 105 to ROI 115. The method can include focusing therapeutic ultrasound energy 120 to create a lesion 25 in a portion of the micro-tears within a tendon in the shoulder 370. The method can include creating a plurality of lesions 25 in the micro-tears within a tendon in the shoulder 370. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within a tendon in the shoulder 370. The method can also include imaging micro-tears within a tendon in the shoulder 370. The method can also include imaging micro-tears within a tendon in the shoulder 370 after the ablating at least a portion of the micro-tears within a tendon in the shoulder 370. The method can include comparing a measurement of the micro-tears within a tendon in the shoulder 370 before and after the ablating step. The method can include directing acoustical pressure or cavitation to the micro-tears within a tendon in the shoulder 370 after the ablating step further improving the micro-tears within a tendon in the shoulder 370. The method can include welding the micro-tears within a tendon in the shoulder 370 with therapeutic ultrasound energy 120. The method can include increasing blood perfusion to the ROI 115. The method can include administering a medicant to the ROI 115. The method can also include any of the steps of method 100.

Various embodiments provide methods for treating a frozen joint. In one embodiment, the method can treat a frozen shoulder. Frozen shoulder, medically referred to as adhesive capsulitis, is a disorder in which the shoulder capsule, the connective tissue surrounding the glenohumeral joint of the shoulder, becomes inflamed and stiff, greatly restricting motion and causing chronic pain.

Such a method of treating a frozen shoulder can include targeting inflamed tissue near or at a portion of a capsule in the shoulder in ROI 115, directing therapeutic ultrasound energy 120 to the inflamed tissue near or at a portion of a capsule in the shoulder, ablating at least a portion of the inflamed tissue near or at a portion of a capsule in the shoulder, and improving the inflamed tissue near or at a portion of a capsule in the shoulder. The method can include coupling ultrasound probe 105 to ROI 115. The method can include focusing therapeutic ultrasound energy 120 to create a lesion 25 in a portion of the inflamed tissue near or at a portion of a capsule in the shoulder. The method can include creating a plurality of lesions 25 in the inflamed tissue near or at a portion of a capsule in the shoulder. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the inflamed tissue near or at a portion of a capsule in the shoulder. The method can also include imaging an inflamed portion of a portion of a capsule in the shoulder. The method can also include imaging the inflamed tissue near or at a portion of a capsule in the shoulder after the ablating at least a portion of the inflamed tissue near or at a portion of a capsule in the shoulder. The method can include comparing a measurement of the inflamed tissue near or at a portion of a capsule in the shoulder before and after the ablating step. The method can include directing acoustical pressure or cavitation to the inflamed tissue near or at a portion of a capsule in the shoulder after the ablating step further improving the inflamed tissue near or at a portion of a capsule in the shoulder. The method can include increasing blood perfusion to the ROI 115. The method can include administering a medicant to the ROI 115. The method can also include any of the steps of method 100.

According to various embodiments, methods of treating a frozen shoulder are provided. Such a method can include targeting micro-tears within a portion of a capsule in the shoulder in ROI 115, directing therapeutic ultrasound energy 120 to the micro-tears within a portion of a capsule in the shoulder, ablating at least a portion of the micro-tears within a portion of a capsule in the shoulder, and improving the micro-tears within a portion of a capsule in the shoulder. The method can include coupling ultrasound probe 105 to ROI 115. The method can include focusing therapeutic ultrasound energy 120 to create a lesion 25 in a portion of the micro-tears within a portion of a capsule in the shoulder. The method can include creating a plurality of lesions 25 in the micro-tears within a portion of a capsule in the shoulder. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within a portion of a capsule in the shoulder. The method can also include imaging micro-tears within a portion of a capsule in the shoulder. The method can also include imaging micro-tears within a portion of a capsule in the shoulder after the ablating at least a portion of the micro-tears within a portion of a capsule in the shoulder. The method can include comparing a measurement of the micro-tears within a portion of a capsule in the shoulder before and after the ablating step. The method can include directing acoustical pressure or cavitation to the micro-tears within a portion of a capsule in the shoulder after the ablating step further improving the micro-tears within a portion of a capsule in the shoulder. The method can include welding the micro-tears within a portion of a capsule in the shoulder with therapeutic ultrasound energy 120. The method can include increasing blood perfusion to the ROI 115. The method can include administering a medicant to the ROI 115. The method can also include any of the steps of method 100.

Of course, the method described above for treating a frozen shoulder can be modified to treat any joint which is restricted in movement by an injured and/or inflamed capsule. For example, various embodiments provide a method for treating an injured capsule in a knee. In another example, various embodiments provide a method for treating an injured capsule in an ankle. In another example, various embodiments provide a method for treating an injured capsule in an elbow. Various embodiments provide a method for treating an injured capsule in any joint in a body.

In various embodiments, a method of treating a hyperextended capsule and/or partially torn capsule can include targeting the hyperextended capsule and/or partially torn capsule in ROI 115, directing therapeutic ultrasound energy 120 to the hyperextended capsule and/or partially torn capsule, ablating at least a portion of the inflamed tissue near or at a portion of a capsule in the shoulder, and improving the inflamed tissue near or at a portion of a capsule in the shoulder. The method can include coupling ultrasound probe 105 to ROI 115. The method can include focusing therapeutic ultrasound energy 120 to create a lesion 25 in a portion of the hyperextended capsule and/or partially torn capsule. The method can include creating a plurality of lesions 25 in the hyperextended capsule and/or partially torn capsule. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the hyperextended capsule and/or partially torn capsule. The method can also include imaging a hyperextended capsule and/or partially torn capsule. The method can include directing acoustical pressure or cavitation to the hyperextended capsule and/or partially torn capsule after the ablating step further improving the hyperextended capsule and/or partially torn capsule hyperextended capsule and/or partially torn capsule. The method can include increasing blood perfusion to the ROI 115. The method can include administering a medicant to the ROI 115. The method can also include any of the steps of method 100.

According to various embodiments, methods of treating a hyperextended capsule and/or partially torn capsule. Such a method can include targeting micro-tears within a portion of a capsule in the shoulder in ROI 115, directing therapeutic ultrasound energy 120 to the micro-tears within a portion of a capsule in the shoulder, ablating at least a portion of the micro-tears within a portion of a capsule in the shoulder, and improving the micro-tears within a portion of a capsule in the shoulder. The method can include coupling ultrasound probe 105 to ROI 115. The method can include focusing therapeutic ultrasound energy 120 to create a lesion 25 in a portion of the micro-tears within a portion of a capsule in the shoulder. The method can include creating a plurality of lesions 25 in the micro-tears within a portion of a capsule in the shoulder. The method can include creating the plurality of lesion 25 in a pattern, such as, a linear pattern, a 2-D pattern, or a 3-D pattern, and combinations thereof. The method further comprising measuring a distance on skin surface 104 and then directing therapeutic ultrasound energy 120 to the micro-tears within a portion of a capsule in the shoulder. The method can also include imaging micro-tears within a portion of a capsule in the shoulder. The method can also include imaging micro-tears within a portion of a capsule in the shoulder after the ablating at least a portion of the micro-tears within a portion of a capsule in the shoulder. The method can include comparing a measurement of the micro-tears within a portion of a capsule in the shoulder before and after the ablating step. The method can include directing acoustical pressure or cavitation to the micro-tears within a portion of a capsule in the shoulder after the ablating step further improving the micro-tears within a portion of a capsule in the shoulder. The method can include welding the micro-tears within a portion of a capsule in the shoulder with therapeutic ultrasound energy 120. The method can include increasing blood perfusion to the ROI 115. The method can include administering a medicant to the ROI 115. The method can also include any of the steps of method 100.

Of course, the method described above for treating a hyperextended capsule and/or partially torn capsule can be modified to treat any joint. For example, various embodiments provide a method for treating a hyperextended capsule and/or partially torn capsule in a knee. In another example, various embodiments provide a method for treating a hyperextended capsule and/or partially torn capsule in an ankle. In another example, various embodiments provide a method for treating a hyperextended capsule and/or partially torn capsule in an elbow. Various embodiments provide a method for treating a hyperextended capsule and/or partially torn capsule in any joint in a body.

The following patents and patent applications are useful for understanding the present disclosure: US Patent Application Publication No. 20050256406, entitled "Method and System for Controlled Scanning, Imaging, and/or Therapy" published November 17, 2005; US Patent Application Publication No. 20060058664, entitled "System and Method for Variable Depth Ultrasound Treatment" published March 16, 2006; US Patent Application Publication No. 20060084891, entitled Method and System for Ultra-High Frequency Ultrasound Treatment" published April 20, 2006; US Patent No. 7,530,958, entitled "Method and System for Combined Ultrasound Treatment" issued May 12, 2009; US Patent Application Publication No. 2008071255, entitled "Method and System for Treating Muscle, Tendon, Ligament, and Cartilage Tissue" published March 20, 2008; US Patent No. 6,623,430, entitled " Method and Apparatus for Safely Delivering Medicants to a Region of Tissue Using Imaging, Therapy, and Temperature Monitoring Ultrasonic System, issued September 23, 2003; US Patent No. 7,571,336, entitled " Method and System for Enhancing Safety with Medical Peripheral Device by Monitoring if Host Computer is AC Powered" issued August 4, 2009; and US Patent Application Publication No. 20080281255, entitled "Methods and Systems for Modulating Medicants Using Acoustic Energy" published November 13, 2008.

Various embodiments and the examples described herein are exemplary and not intended to be limiting the invention as defined in the appended claims.

## Claims

1. A system (100) for treating an injury in fibrous soft tissue, the system (100) comprising: a hand-held probe (102) comprising:
a housing containing:
an ultrasound transducer (75) configured to focus a conformal distribution of ultrasound energy into a region of interest (ROI) comprising a portion of an injury in fibrous soft tissue;
a position sensor (107) configured to communicate at least one of a position of the housing and a speed of movement of the housing;
a tissue contact sensor configured to communicate a coupling of the transducer (75) to the region of interest comprising a portion of the injured fibrous soft tissue;
a communication interface configured for communication with the ultrasound transducer (75), the position sensor (107), and the tissue contact sensor; and
a rechargeable power supply configured to supply power to the ultrasound transducer (75), the position sensor (107), tissue contact sensor, and the communication interface;
a controller (144) in communication with the communication interface and configured to control a spatial parameter and a temporal parameter of the ultrasound transducer (75) to emit the conformal distribution of ultrasound energy,
wherein the controller (144) is configured to receive the at least one of the position of the housing and the speed of movement of the housing, and is configured to control the timing of conformal distribution of ultrasound energy based on at least one of the position and the speed,
wherein controlling the timing of the conformal distribution of ultrasound energy comprises adjustment and optimization of drive amplitude levels, frequency, and waveform selections of the types of pulses.

2. The system according to claim 1, wherein the housing contains a motion mechanism (77) coupled to the ultrasound transducer (75) and configured to scan the ultrasound transducer (75) in at least one of a linear pattern and a two-dimensional pattern and the controller (144) is configured to receive the at least one of the position of the housing and the speed of movement of the housing, and is configured to control the scan of the motion mechanism (77) based on at least one of the position and the speed.

3. The system according to claim 1, further comprising a second energy source contained in the housing and configured to direct a second energy into the region of interest (ROI) comprising the injury in a portion of the fibrous soft tissue.

4. The system according to claim 3, wherein the second energy source is one of a laser, an intense pulsed light, a light emitting diode, a radiofrequency generator, photon-based energy source, plasma source, a magnetic resonance source, and a mechanical energy source.

5. The system according to claim 1, wherein the fibrous soft tissue comprises at least a portion of a ligament (138).

6. The system according to claim 1, wherein the fibrous soft tissue comprises at least a portion of a tendon.

7. The system according to claim 1, wherein the fibrous soft tissue comprises at least a portion of fascia (204).

8. The system according to claim 1, wherein the fibrous soft tissue comprises at least a portion of an Achilles tendon and further comprising treating at least one of a strain and a partial tear in the Achilles tendon.

9. The system according to claim 1, wherein the fibrous soft tissue comprises at least a portion of a tendon of extensor of carpi radialis brevis muslien and further comprising treating tennis elbow.

## Patentansprüche

1. Ein System (100) zur Behandlung einer Verletzung in faserigem Weichgewebe, wobei das System (100) Folgendes umfasst: eine handgehaltene Sonde (102), die Folgendes umfasst:
ein Gehäuse, enthaltend:
einen Ultraschallwandler (75), der so konfiguriert ist, dass er eine konforme Verteilung von Ultraschallenergie in einen Bereich von Interesse (ROI) fokussiert, der einen Teil einer Verletzung in faserigem Weichgewebe umfasst;
einen Positionssensor (107), der so konfiguriert ist, dass er mindestens eines von: einer Position des Gehäuses und einer Bewegungsgeschwindigkeit des Gehäuses übermittelt;
einen Gewebekontaktsensor, der so konfiguriert ist, dass er eine Kopplung des Wandlers (75) mit dem Bereich von Interesse, der einen Teil des verletzten faserigen Weichgewebes umfasst, übermittelt;
eine Kommunikationsschnittstelle, die für die Kommunikation mit dem Ultraschallwandler (75), dem Positionssensor (107) und dem Gewebekontaktsensor konfiguriert ist; und
eine wiederaufladbare Stromversorgung, die so konfiguriert ist, dass sie den Ultraschallwandler (75), den Positionssensor (107), den Gewebekontaktsensor und die Kommunikationsschnittstelle mit Strom versorgt;
ein Steuergerät (144), das mit der Kommunikationsschnittstelle in Verbindung steht und so konfiguriert ist, dass es einen räumlichen Parameter und einen zeitlichen Parameter des Ultraschallwandlers (75) steuert, um die konforme Verteilung von Ultraschallenergie auszusenden,
wobei das Steuergerät (144) so konfiguriert ist, dass es das mindestens eine von: der Position des Gehäuses und der Bewegungsgeschwindigkeit des Gehäuses empfängt und so konfiguriert ist, dass es das Timing der konformen Verteilung der Ultraschallenergie auf der Grundlage von mindestens einem von: der Position und der Geschwindigkeit steuert,
wobei die Steuerung des Timings der konformen Verteilung der Ultraschallenergie die Einstellung und Optimierung der Ansteuerungsamplitudenpegel, der Frequenz, und der Auswahl der Wellenform der Impulstypen umfasst.

2. Das System nach Anspruch 1, wobei das Gehäuse einen Bewegungsmechanismus (77) enthält, der mit dem Ultraschallwandler (75) verbunden und so konfiguriert ist, dass er den Ultraschallwandler (75) in mindestens einem von: einem linearen Muster und einem zweidimensionalen Muster abtastet, und das Steuergerät (144) so konfiguriert ist, dass das mindestens eine von: der Position des Gehäuses und der Bewegungsgeschwindigkeit des Gehäuses empfängt und die Abtastung des Bewegungsmechanismus (77) auf der Grundlage von mindestens einem von: der Position und der Geschwindigkeit steuert.

3. Das System nach Anspruch 1, das ferner eine zweite Energiequelle umfasst, die im Gehäuse enthalten und so konfiguriert ist, dass sie eine zweite Energie in den Bereich von Interesse (ROI) leitet, der die Verletzung in einem Teil des faserigen Weichgewebes umfasst.

4. Das System nach Anspruch 3, wobei die zweite Energiequelle eines von: einem Laser, einem intensiv gepulsten Licht, einer lichtemittierenden Diode, einem Hochfrequenzgenerator, einer photonenbasierten Energiequelle, einer Plasmaquelle, einer magnetischen Resonanzquelle und einer mechanischen Energiequelle ist.

5. Das System nach Anspruch 1, wobei das faserige Weichgewebe mindestens einen Teil eines Bandes (138) umfasst.

6. Das System nach Anspruch 1, wobei das faserige Weichgewebe mindestens einen Teil einer Sehne umfasst.

7. Das System nach Anspruch 1, wobei das faserige Weichgewebe mindestens einen Teil einer Faszie (204) umfasst.

8. Das System nach Anspruch 1, wobei das faserige Weichgewebe mindestens einen Teil einer Achillessehne umfasst und ferner die Behandlung von mindestens einem von: einer Zerrung und einem Teilriss in der Achillessehne umfasst.

9. Das System nach Anspruch 1, wobei das faserige Weichgewebe mindestens einen Teil einer Sehne des Musculus extensor carpi radialis brevis umfasst und ferner die Behandlung des Tennisellenbogens umfasst.

## Revendications

1. Système (100) pour traiter une blessure dans du tissu mou fibreux, le système (100) comprenant : une sonde tenue à la main (102) comprenant :
un boîtier contenant :
un transducteur à ultrasons (75) configuré pour focaliser une distribution conformée d'énergie ultrasonore dans une région d'intérêt (ROI) comprenant une portion d'une blessure dans du tissu mou fibreux ;
un capteur de position (107) configuré pour communiquer au moins un élément parmi une position du boîtier et une vitesse de déplacement du boîtier ;
un capteur de contact tissulaire configuré pour communiquer un couplage du transducteur (75) avec la région d'intérêt comprenant une portion du tissu mou fibreux blessé ;
une interface de communication configurée pour une communication avec le transducteur à ultrasons (75), le capteur de position (107) et le capteur de contact tissulaire ; et
une alimentation rechargeable configurée pour fournir une alimentation au transducteur à ultrasons (75), au capteur de position (107), au capteur de contact tissulaire et à l'interface de communication ;
une commande (144) en communication avec l'interface de communication et configurée pour commander un paramètre spatial et un paramètre temporel du transducteur à ultrasons (75) pour émettre la distribution conformée d'énergie ultrasonore,
dans lequel la commande (144) est configurée pour recevoir le au moins un élément parmi la position du boîtier et la vitesse de déplacement du boîtier, et est configurée pour commander le cadencement de la distribution conformée d'énergie ultrasonore sur la base d'au moins un élément parmi la position et la vitesse,
dans lequel la commande du cadencement de la distribution conformée d'énergie ultrasonore comprend le réglage et l'optimisation de niveaux d'amplitude de commande, de la fréquence et de sélections de forme d'onde des types d'impulsions.

2. Système selon la revendication 1, dans lequel le boîtier contient un mécanisme de mouvement (77) couplé au transducteur à ultrasons (75) et configuré pour balayer le transducteur à ultrasons (75) selon au moins un motif parmi un motif linéaire et un motif bidimensionnel, et la commande (144) est configurée pour recevoir le au moins un élément parmi la position du boîtier et la vitesse de déplacement du boîtier, et est configurée pour commander le balayage du mécanisme de mouvement (77) sur la base d'au moins un élément parmi la position et la vitesse.

3. Système selon la revendication 1, comprenant en outre une seconde source d'énergie contenue dans le boîtier et configurée pour diriger une seconde énergie dans la région d'intérêt (région of interest, ROI) comprenant la blessure dans une portion du tissu mou fibreux.

4. Système selon la revendication 3, dans lequel la seconde source d'énergie est un élément parmi un laser, une lumière pulsée intense, une diode électroluminescente, un générateur de radiofréquence, une source d'énergie à base de photons, une source de plasma, une source de résonance magnétique et une source d'énergie mécanique.

5. Système selon la revendication 1, dans lequel le tissu mou fibreux comprend au moins une portion d'un ligament (138).

6. Système selon la revendication 1, dans lequel le tissu mou fibreux comprend au moins une portion d'un tendon.

7. Système selon la revendication 1, dans lequel le tissu mou fibreux comprend au moins une portion d'un fascia (204).

8. Système selon la revendication 1, dans lequel le tissu mou fibreux comprend au moins une portion d'un tendon d'Achille et comprend en outre le traitement d'au moins un élément parmi une foulure et une déchirure partielle du tendon d'Achille.

9. Système selon la revendication 1, dans lequel le tissu mou fibreux comprend au moins une portion du muscle court extenseur radial du carpe et comprend en outre le traitement d'une épicondylite latérale.
